(19) Europäisches Patentamt
European Patent Office
Office européen des brevets

(11) Numéro de publication: **0 287 468 B1**

(12) # FASCICULE DE BREVET EUROPEEN

(45) Date de publication de fascicule du brevet: **10.03.93**

(51) Int. Cl.⁵: **C07D 471/22**, A61K 31/495, C07D 209/14, C07D 403/06, C07D 471/14, //(C07D471/22, 241:00,221:00,221:00,209:00), (C07D471/14,241:00,221:00, 209:00)

(21) Numéro de dépôt: **88400901.0**

(22) Date de dépôt: **14.04.88**

(54) **Nouveaux dérivés 17-aza de 20,21-dinoréburnaménine, leur procédé et les nouveaux intermédiaires de préparation, leur application comme médicaments et les compositions les renfermant.**

(30) Priorité: **15.04.87 FR 8705361**

(43) Date de publication de la demande:
**19.10.88 Bulletin 88/42**

(45) Mention de la délivrance du brevet:
**10.03.93 Bulletin 93/10**

(84) Etats contractants désignés:
**AT BE CH DE ES FR GB GR IT LI LU NL SE**

(56) Documents cités:
**FR-A- 2 168 853**

**CHEMICAL ABSTRACTS, vol. 81, 1974, page 498, abrégé no. 105181k, Columbus, Ohio, US; A. NEKLYUDOV et al.: "Synthesis and pharmacological activity of amino alkyl derivatives of substituted tryptamines", & KHIM.-FARM. ZH. 1974, 8(6), 7-11**

(73) Titulaire: **ROUSSEL-UCLAF**
**35, Boulevard des Invalides**
**F-75007 Paris(FR)**

(72) Inventeur: **Aktogu, Nurgün**
**2, rue Edmond About**
**F-92350 Le Plessis Robinson(FR)**
Inventeur: **Clemence François**
**2, rue Turgot**
**F-75009 Paris(FR)**

(74) Mandataire: **Bourgouin, André et al**
**Département des Brevets ROUSSEL UCLAF**
**111, route de Noisy B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 287 468 B1

## Description

La présente invention concerne de nouveaux dérivés 17-aza de 20,21-dinoréburnaménine, leur procédé de préparation,leur application comme médicaments, les compositions les renfermant, et les nouveaux intermédiaires obtenus lors de leur préparation.

L'invention a pour objet de nouveaux composés de formule (I) :

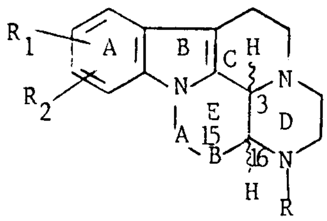

(I)

dans laquelle $R_1$ et $R_2$, identiques ou différents, représentent un atome d'hydrogène, un atome d'halogène, un radical alcoyle renfermant de 1 à 5 atomes de carbone, alcoxy renfermant de 1 à 5 atomes de carbone, un radical hydroxy, trifluorométhyle ou nitro, R représente un atome d'hydrogène, un radical alcoyle renfermant de 1 à 5 atomes de carbone, un radical arylalcoyle renfermant de 7 à 12 atomes de carbone, un radical benzoyle ou alcoylcarbonyle dans lequel le radical alcoyle renferme de 1 à 12 atomes de carbone, et dans laquelle le groupement :

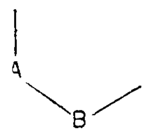

représente soit

i) 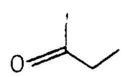

soit

ii) 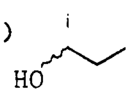

soit

iii) 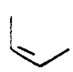

soit

iiii) 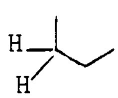

lesdits produits de formule (I) étant sous toutes les formes isomères possibles racémiques ou optiquement actives, ainsi que les sels d'addition avec les acides minéraux ou organiques desdits produits de formule (I).

Dans les produits de formule (I), l'atome d'hydrogène en position 3 et l'atome d'hydrogène en position 16, peuvent chacun occuper l'une ou l'autre des orientations alpha et béta, ce qui détermine l'existence de diastéréoisomères cis et trans. De même, le radical hydroxy en position 14 peut être sous la forme alpha ou béta.

Lorsque $R_1$, $R_2$ et R représentent un radical alcoyle, il s'agit de préférence des radicaux méthyle, éthyle, n-propyle ou isopropyle mais ces substituants peuvent également représenter un radical n-butyle, isobutyle, n-pentyle.

Lorsque $R_1$ et $R_2$ représentent un radical alkoxy, il s'agit de préférence d'un radical méthoxy ou éthoxy, mais ils peuvent aussi représenter un radical propoxy, isopropoxy, butoxy linéaire, secondaire ou tertiaire.

Lorsque $R_1$ et $R_2$ représentent un atome d'halogène, il s'agit de préférence de l'atome de chlore, mais ils peuvent aussi représenter un atome de fluor, de brome ou d'iode.

Lorsque R représente un radical arylalcoyle renfermant de 7 à 12 atomes de carbone, il s'agit de préférence d'un radical benzyle ou phénéthyle.

Lorsque R représente un radical alcoylcarbonyle, il s'agit de préférence d'un radical acétyle ou lauroyle.

La présente invention a en particulier pour objet les composés de formule (I), caractérisés en ce que que $R_1$ et $R_2$ représentent un atome d'hydrogène, un radical méthyle, éthyle, propyle, méthoxy ou éthoxy, un atome de chlore, un radical hydroxy, trifluorométhyle ou nitro, et R représente un atome d'hydrogène, un radical méthyle, éthyle ou propyle, benzyle, phénéthyle, ou benzoyle, sous toutes les formes isomères possibles racémiques ou optiquement actives, ainsi que les sels d'addition avec les acides minéraux ou organiques.

L'invention concerne aussi plus particulièrement les composés de formule (I) caractérisés en ce que $R_2$ représente un atome d'hydrogène, $R_1$ représente un atome d'hydrogène, un radical méthoxy ou éthoxy, un atome de chlore sous toutes les formes isomères possibles racémiques ou optiquement actives, ainsi que les sels d'addition avec les acides minéraux ou organiques et ceux caractérisés en ce que l'atome d'hydrogène en position 3 et l'atome d'hydrogène en position 16 sonttrans sous toutes les formes isomères possibles racémiques ou optiquement actives ainsi que les sels d'addition avec les acides minéraux ou organiques.

L'invention a tout particulièrement pour objet les produits décrits ci-après dans les exemples et spécialement :

- la (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine,

et leurs sels d'addition avec les acides.

Les sels d'addition avec les acides minéraux ou organiques des produits de formule I peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques, tel que l'acide méthanesulfonique, l'acide éthane sulfonique, l'acide propane sulfonique, les acides alcoyldisulfoniques tels que l'acide méthanedisulfonique, l'acide alpha, béta-éthanedisulfonique, les acides arylmonosulfoniques, tel que l'acide benzènesulfonique et les acides aryldisulfoniques.

L'invention a aussi pour objet un procédé de préparation des composés de formule (I) caractérisé en ce que l'on fait réagir un composé de formule (II) :

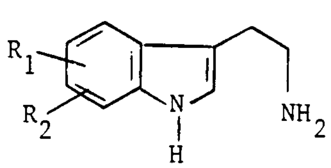

(II)

dans laquelle $R_1$ et $R_2$ ont les significations données ci-dessus avec le chloracétonitrile de manière à obtenir un composé de formule (III)

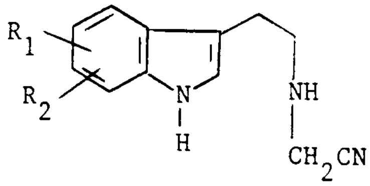

(III)

que l'on réduit en un composé de formule (IV) :

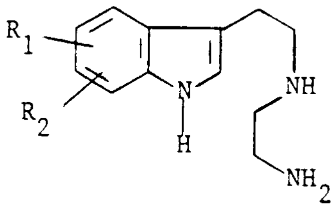

(IV)

que l'on condense avec un maléate ou un fumarate d'alcoyle de formule

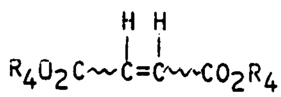

dans laquelle $R_4$ est un radical alcoyle renfermant de 1 à 4 atomes de carbone pour obtenir un composé de formule (V) :

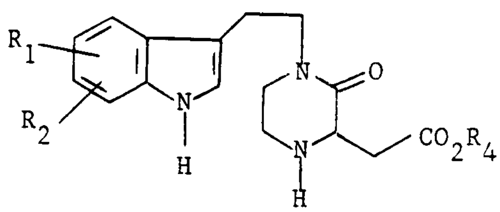

(V)

sur lequel on fait réagir un réactif susceptible d'introduire un radical R', R' ayant les valeurs indiquées pour R à l'exception de la valeur hydrogène, pour obtenir un composé de formule (VI) :

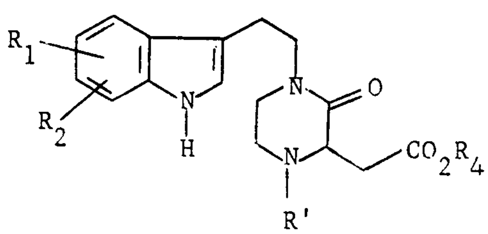

(VI)

sur lequel on fait réagir un agent de cyclisation pour obtenir un composé de formule (VII) :

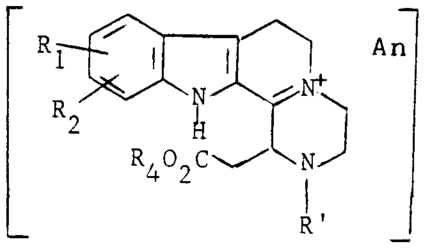

(VII)

dans lequel $R_1$, $R_2$, $R_4$ et R' ont les significations indiquées précédemment et An représente un anion dérivé d'un acide fort et que, soit l'on réduit le composé de formule (VII) en composé de formule (VIII) dans laquelle les deux atomes d'hydrogène en position 3 et 16 sont en position cis :

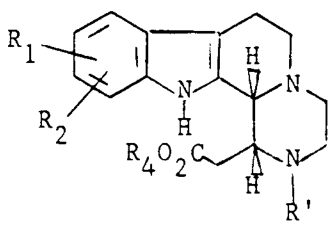

(VIII)

dont on élimine le cas échéant le groupement R' dans le cas où celui-ci représente un radical benzoyle ou alcoylcarbonyle et que l'on cyclise pour obtenir un composé de formule

$(I_{A_{1b}})$

correspondant à un produit de formule (I) dans laquelle :

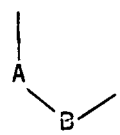

représente

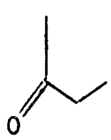

les deux atomes d'hydrogène en position 3 et 16 sont en position cis et R représente un atome d'hydrogène et composé de formule

$(I_{A_{1b}})$

que l'on soumet le cas échéant à l'action d'un réactif susceptible d'introduire un radical R', R' ayant toutes les valeurs indiquées pour R à l'exception de la valeur hydrogène, pour obtenir un composé de formule

$(I_{A_{1c}})$

dans laquelle R a la signification indiquée ci-dessus pour R', soit l'on cyclise le composé de formule (VII) en composé de formule (IX) :

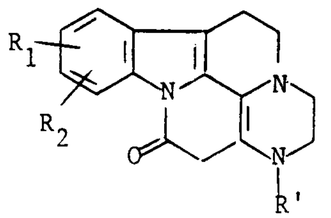

(IX)

que l'on réduit pour obtenir un composé de formule

$(I_{A_{2a}})$

correspondant à un produit de formule (I) dans laquelle :

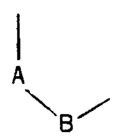

représente

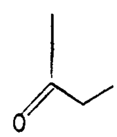

les deux atomes d'hydrogène en position 3 et 16 sont en position trans et R a la signification indiquée ci-dessus pour R', dont on élimine le cas échéant, le groupement R' dans le cas où celui-ci représente un radical benzoyle ou alcoylcarbonyle, pour obtenir un composé de formule

$(I_{A_{2b}})$

dans laquelle R est un atome d'hydrogène, que l'on soumet, le cas échéant, à l'action d'un réactif susceptible d'introduire un radical R', R' ayant les valeurs indiquées pour R à l'exception de la valeur hydrogène pour obtenir un composé de formule

$(I_{A_{2c}})$

dans laquelle R a la signification indiquée ci-dessus pour R', lesdits composés de formules

$(I_{A_{1b}})$ ,

$(I_{A_{1c}})$, $(I_{A_{2a}})$, $(I_{A_{2b}})$ et $(I_{A_{2c}})$

étant, si désiré, réduits en composés correspondants de formules

$(I_{B_{1b}})$, $(I_{B_{1c}})$, $(I_{B_{2a}})$,

$(I_{B_{2b}})$ et $(I_{B_{2c}})$

représentant les composés de formule (I) dans laquelle :

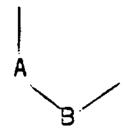

représente

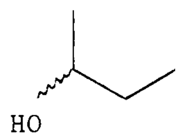

lesdits composés de formules

$(I_{B_{1b}})$, $(I_{B_{1c}})$, $(I_{B_{2a}})$,

$(I_{B_{2b}})$, $(I_{B_{2c}})$

étant, si désiré, soumis à l'action d'un agent déshydratant pour obtenir les composés correspondants de formules

$(I_{C_{1b}})$, $(I_{C_{1c}})$, $(I_{C_{2a}})$, $(I_{C_{2b}})$ et $(I_{C_{2c}})$

représentant les composés de formule I dans laquelle

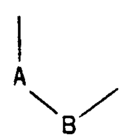

: représente

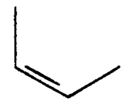

lesdits composés de formules

$(I_{C_{1b}})$, $(I_{C_{1c}})$, $(I_{C_{2a}})$,

$(I_{C_{2b}})$ et $(I_{C_{2c}})$

étant, si désiré, soumis à l'action d'un agent de réduction, pour obtenir les composés correspondants de formules

$(I_{D_{1b}})$,

$(I_{D_{1c}})$, $(I_{D_{2a}})$, $(I_{D_{2b}})$ et $(I_{D_{2c}})$

eprésentant les composés de formule (I) dans laquelle :

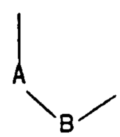

représente

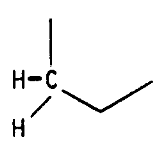

et traite si désiré tous les produits de formule (I) obtenus par un acide minéral ou organique pour en former les sels.

Dans des conditions préférentielles de mise en oeuvre de l'invention, le procédé ci-dessus est réalisé de la manière suivante :

- La transformation du composé de formule (II) en composé de formule (III) est effectuée par la condensation du chloroacétonitrile en milieu basique.
- La base utilisée est de préférence le carbonate de potassium, soit en présence d'hydrogénosulfate de tétrabutylammonium, soit dans un solvant aprotique tel que le diméthylformamide. On peut aussi utiliser la triéthylamine dans le tétrahydrofuranne.

- La méthode de réduction utilisée pour obtenir le composé de formule (IV) est l'hydrogénation catalytique en présence de nickel de Raney.
- On condense le composé de formule (IV) avec le diéthylmaléate dans l'éthanol au reflux.
- Le réactif susceptible d'introduire R' dans les composés de formule (V),

$$(I_{A_{1b}}) \text{ ou } (I_{A_{2b}})$$

est

- soit le mélange aldéhyde formique/acide acétique si l'on souhaite obtenir un composé de formule (VI),

$$(I_{A_{1c}}) \text{ ou } (I_{A_{2c}})$$

dans laquelle R' représente un radical méthyle ;

- soit un composé de formule X-R' dans laquelle X représente n atome d'halogène et R' a la signification indiquée ci-dessus pour R à l'exception de la valeur hydrogène. Ainsi, X est notamment un atome d'iode lorsque R' est un radical alcoyle et l'on opère alors notamment en présence de carbonate de potassium et d'hydrogénosulfate de tétrabutylammonium, un atome de chlore lorsque R' est un radical benzoyle ou alcoycarbonyle et un atome de brome lorsque R' est un radical aralcoyle.

On opère par exemple en présence d'une base azotée telle que la triéthylamine ou d'une base minérale telle qu'un carbonate alcalin au sein d'un solvant organique tel que le tétrahydrofuranne ou le diméthylformamide.

Dans le cas où l'on utilise un halogénure peu réactif ou encombré, il peut être intéressant d'opérer en présence d'un sel de sodium tel que l'iodure.

Dans des conditions très préférentielles, la méthylation du composé de formule

$$(I_{A_{2b}})$$

s'effectue avec de l'iodure de méthyle en présence de carbonate de potassium et d'hydrogènosulfate de tétrabutylammonium ou avec le mélange acide formique/aldéhyde formique, alors que la méthylation du composé de formule

$$(I_{A_{1b}})$$

est réalisée avec le mélange acide formique/aldéhyde formique.

- La cyclisation du composé de formule (VI) en composé de formule (VII) est effectuée par action de l'oxychlorure de phosphore puis de l'acide iodhydrique ou de l'acide perchlorique, l'anion $An^-$ étant dans ce cas, le reste de cet acide.
- La réduction du composé de formule (VII) en composé de formule (VIII) s'effectue avec du borohydrure de sodium.
- L'élimination éventuelle du groupement R' du composé de formule (VIII) et la cyclisation de ce composé sont effectuées simultanément en milieu acide et de préférence en présence d'acide chlorhydrique.
- La cyclisation du composé de formule (VII) en composé de formule (IX) est réalisée en milieu basique et de préférence en milieu ammoniacal.
- La réduction du composé de formule (IX) en composé de formule

$$(I_{A_{2a}})$$

s'effectue avec du borohydrure de sodium, en présence d' acide acétique.

- L'élimination éventuelle du groupement R' des composés de formule

$$(I_{A_{2a}})$$

est effectuée par exemple avec un acide tel que l'acide chlorhydrique.

- La réduction des composés de formule ($I_A$) dans laquelle :

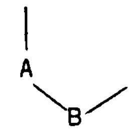

représente

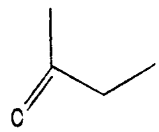

en composés de formule ($I_B$) dans laquelle :

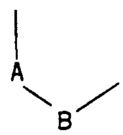

représente

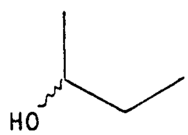

est réalisée avec un hydrure, notamment un hydrure mixte, tel que par exemple, l'hydrure mixte delithium et d'aluminium, le diéthylhydrure de sodium et d'aluminium.

- L'agent de déshydratation utilisé pour obtenir à partir des composés de formule ($I_B$) dans laquelle :

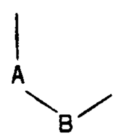

représente

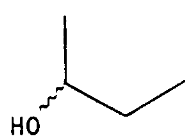

des composés de formule ($I_C$) dans laquelle :

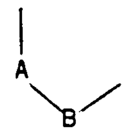

représente

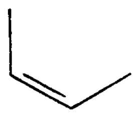

est un acide tel tel que l'acide chlorhydrique, l'acide bromhydrique, l'acide sulfurique, l'acide acétique, l'acide paratoluène sulfonique, l'acide méthanesulfonique.

- L'agent de réduction auxquels sont soumis les composés de formule ($I_C$) dans laquelle

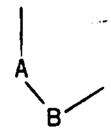

représente

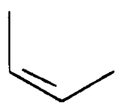

pour obtenir les composés de formule ($I_D$) dans laquelle

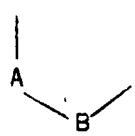

représente

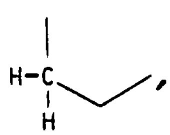

est l'hydrogène en présence d'un catalyseur tel que le platine, le palladium.

- Les formes optiquement actives des produits de formule (I) peuvent être préparés par dédoublement des racémiques, selon les méthodes usuelles.

Les composés de formule (I) tels que définis ci-dessus ainsi que leurs sels d'addition avec les acides présentent d'intéressantes propriétés pharmacologiques. Ils présentent en particulier une très bonne activité analgésique.

Ils sont aussi des oxygénateurs et vasorégulateurs cérébraux intéressants.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémique ou optiquement actives, ainsi que les sels d'addition avec les acides minéraux ou organiques pharmaceutiquement acceptables desdits produits de formule (I).

L'invention a plus particulièrement pour objet, à titre de médicaments :

- la (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine, et leurs sels d'addition avec les acides pharmaceutiquement acceptables.

Les médicaments, objet de l'invention, peuvent être utilisés dans le traitement des algies musculaires, articulaires ou nerveuses, les douleurs dentaires, les migraines, le zona et également à titre de traitement complémentaire dans les états infectieux et fébriles.

Ils peuvent être aussi utilisés dans le traitement des vasculopathies cérébrales et de tous les syndromes provoqués par une altération de la circulation cérébrale.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 20 mg à 2 g par jour chez l'adulte, par voie orale.

La présente invention a enfin pour objet à titre de produits industriels nouveaux, notamment à titre de produits intermédiaires nécessaires à la préparation des produits de formule (I), les produits de formules (III), (IV') correspondant à la formule (IV) dans laquelle lorsque l'un de $R_1$ ou $R_2$ représente un atome d'hydrogène, l'autre de $R_1$ ou $R_2$ ne représente pas un radical méthoxy, les composés (V), (VI), (VII), (VIII) et (IX).

Les exemples donnés ci-après illustrent l'invention, sans toutefois la limiter.

**Exemple 1 : dichlorhydrate de la (±) 20,21-dinor 17-aza-éburnaménin 14(15H)-one**

**Stade A :** [[2-(1H-indol 3-yl) éthyl] amino] acétonitrile.

A une suspension de 1 g de tryptamine dans 10 cm3 de dichlorométhane, on ajoute 5 cm3 d'eau, 0,856 g de carbonate de potassium, 0,210 g de sulfate de n-tétrabutylammonium et 0,44 cm3 de chlorure d'acétonitrile et laisse sous agitation pendant 48 heures et sous atmosphère inerte à température ambiante. Ensuite on décante, lave la phase organique à l'eau, sèche, évapore le solvant et chromatographie le résidu sur silice en éluant par un mélange chlorure de méthylène - acétate d'éthyle (7-3). On obtient 0,861 g de produit attendu.

Spectre IR ($CHCl_3$) :

| | |
|---|---|
| NH indole | 3 480 $cm^{-1}$ |
| l'autre NH | 3 327 $cm^{-1}$ |
| CN ~ | 2 240 $cm^{-1}$ |
| | 2 225 $cm^{-1}$ |
| système conjugué aromatique | 1 618 $cm^{-1}$ |
| | 1 596 $cm^{-1}$ |
| | 1 552 $cm^{-1}$ |

**Stade B :** 2-(1H-indol 3-yl) éthyléthane diamine.

On hydrogène une solution de 5 g du produit obtenu ci-dessus dans 100 cm3 de méthanol en présence de 15 g de nickel de Raney, pendant 1 heure 30 minutes à température ambiante. On filtre et concentre à sec le filtrat. On reprend le résidu par du chlorure de méthylène, sèche et amène à sec. On empâte avec du pentane la résine obtenue, plusieurs fois et obtient 4,95 g de produit attendu sous forme de base.

**Stade C :** 4-[2-(1H-indol 3-yl) éthyl] 3-oxo 2-pipérazine acétate d'éthyle.

On porte au reflux 36 g de produit obtenu comme ci-dessus dans 250 cm3 d'éthanol et on introduit à chaud pendant 5 heures 30 minutes, 47 cm3 de maléate de diéthyle dans 50 cm3 d'éthanol. On maintient le reflux encore 1 heure, puis amène à sec et chromatographie sous pression sur silice le résidu huileux obtenu, en éluant par le mélange acétate d'éthyle-éthanol (9-1). On récupère 23,1 g de produit attendu F = 80¤C.

**Stade D :** 1-benzoyl 4-[2-(1H-indol 3-yl) éthyl] 3-oxo 2-pipérazine acétate d'éthyle.

On dissout 22,4 g du produit obtenu en C dans 225 cm3 de tétrahydrofuranne et 14,3 cm3 de triéthylamine. On refroidit à 5¤C, afin d'y ajouter lentement 7,9 cm3 de chlorure de benzoyle, puis maintient l'agitation pendant 1 heure à température ambiante. On essore l'insoluble, amène à sec le filtrat et obtient 28,4 g de produit attendu F = 144¤C.

**Stade E :** Chlorhydrate de cis (±) 2-benzoyl 1, 2, 3, 4, 6, 7, 12, 12 b - octahydro pyrazono/1',2'-1,2/pyrido [3,4-b] indole 1-acétate d'éthyle.

On chauffe à 90¤C pendant 2 heures 11,4 g du produit précédent dans 40 cm3 d'oxychlorure de phosphore. On amène à sec, reprend le résidu par 50 cm3 d'acétone, verse la solution lentement sur un mélange de 30 cm3 d'acide iodhydrique à 56 % et 300 cm3 d'eau glacée, puis laisse sous agitation à 0¤C pendant 1 heure. On essore le précipité, sèche et ajoute 150 cm3 de méthanol en agitant, puis on introduit par petites fractions 2,5 g de borhydrure de sodium et maintient l'agitation pendant 30 minutes. On ajoute 10 cm3 d'acide acétique, agite 1/4 d'heure, ajoute 20 cm3 d'ammoniaque et finalement 200 cm3 d'eau glacée. On essore le précipité formé, lave à l'eau, sèche à 80¤C sous pression réduite, puis chromatographie sur silice le produit obtenu (éluant : chlorure de méthylène - acétate d'éthyle 5-5) pour isoler 7,25 g de produit attendu sous forme de base F = 260¤C.

A partir de 3,4 g de base dans 100 cm3 d'éthanol et 100 cm3 d'acétate d'éthyle avec 0,69 cm3 d'acide chlorhydrique 11,8 N, on prépare 3 g du chlorhydrate attendu.

Spectre IR de la base ($CHCl_3$) :

= C - NH - à 3 459 $cm^{-1}$

NH et/ou OH associé 3 223 et 3 204 $cm^{-1}$ (doublet)

>C = O { ester : 1 722 $cm^{-1}$ ; amide : 1 624 $cm^{-1}$ }

aromatique : { 1 602 $cm^{-1}$ ; 1 577 $cm^{-1}$ ; 1 495 $cm^{-1}$ }

**Stade F :** Dichlorhydrate de la (±) 20, 21-dinor 17-aza éburnaménin 14 (15 H)-one.

On porte au reflux pendant 21 heures 19,6 g de produit obtenu au stade E sous forme de base dans 250 cm3 d'acide chlorhydrique 5 N, traite au charbon actif, filtre et alcalinise par addition d'ammoniaque concentrée. On extrait au chlorure de méthylène, lave à l'eau, sèche, concentre à sec et chromatographie sur silice (éluant : chlorure de méthylène-méthanol 9-1). On isole 7 g de produit sous forme de base F = 200¤C.

On dissout 3 g de base dans 100 cm3 d'acétate d'éthyle et 100 cm3 d'isopropanol, ajoute 1,9 cm3 d'acide chlorhydrique 11,8 N, essore, lave à l'acétate d'éthyle et à l'éthanol et obtient 3,52 g de produit brut

que l'on purifie par empâtage à chaud dans l'éthanol chaud. On obtient 3,2 g de dichlorhydrate attendu.

Spectre IR (base) ($CHCl_3$) :

| | |
|---|---|
| C = 0 | 1 704 $cm^{-1}$ |
| C = C | 1 632 $cm^{-1}$ |
| + aromatique | 1 602 $cm^{-1}$ |
| | 1 473 $cm^{-1}$ |
| type NH | 3 340 $cm^{-1}$ |

**Exemple 2 : Dichlorhydrate de la (±) 17-(2-phényléthyl) 17-aza 20,21-dinor-éburnaménin 14 (15 H)-one**

On dissout à 60¤C 4,37 g d'iodure de sodium dans 50 cm3 de diméthylformamide, ajoute sous agitation dans ces conditions, 3,9 cm3 de bromure de 2-phényléthyle. Après une demi-heure, on introduit 2,6 g de (±) 17-aza 20,21-dinor-éburnaménin 14 (15 H)-one, puis 4,03 g de carbonate de potassium. On agite à 60¤C pendant 72 heures et laisse revenir à température ambiante, ajoute 100 cm3 d'eau et extrait au chlorure de méthylène. On lave la phase organique à l'eau jusqu'à neutralité, sèche, amène à sec, chromatographie le résidu sur silice (éluant : chlorure de méthylène-méthanol 95-5) et obtient 900 mg de produit attendu sous forme de base F = 200¤C.

On dissout 1,55 g de base obtenue comme ci-dessus dans 100 cm3 d'un mélange acétate d'éthyle-éthanol (1-1), ajoute 0,71 cm3 d'acide chlorhydrique 11,8 N et agite pendant 4 heures. On essore le précipité, lave à l'éthanol, puis à l'acétate d'éthyle et obtient 1,65 g de produit attendu F = 220¤C.

**Exemple 3 : Maléate neutre de la (±) 17-méthyl 17-aza 20,21-dinor-éburnaménin 14 (15H)-one.**

On mélange 4,26 g de (±) 20,21-dinor 17-azaéburnaménin 14 (15 H)-one, 17 cm3 d'aldéhyde formique à 40 % et 11 cm3 d'acide formique, chauffe à 70¤C pendant 3 heures 30 minutes. On verse le mélange réactionnel sur de l'eau et de la glace, alcalinise par addition d'ammoniaque et extrait au chlorure de méthylène. On lave la phase organique à l'eau, sèche, amène à sec et chromatographie le résidu sur silice (éluant : chlorure de méthylène-méthanol 93-7). On obtient 2,55 g de produit sous forme de base.

On dissout 2,1 g de ce dernier dans 100 cm3 d'acétate d'éthyle et 50 cm3 d'éthanol et ajoute 867 mg d'acide maléique, puis agite 2 heures 30 minutes. On essore, lave le précipité avec de l'acétate d'éthyle, puis de l'éthanol et obtient 2,6 g de produit attendu F = 255¤C.

**Exemple 4 : (±) 17-benzoyl 14, 15-dihydro 14-oxo 17-aza 20,21-dinor-éburnaménine (chlorhydrate)**

On dissout 2,5 g de (±) 20, 21-dinor 17-aza éburnaménin 14 (15H)-one dans 80 cm3 de tétrahydrofuranne et 2 cm3 de triéthylamine, refroidit à 10¤C, afin d'ajouter lentement 1,1 cm3 de chlorure de benzoyle en maintenant la température à 10-15¤C, puis on agite encore une heure à température ambiante. On essore le précipité, amène à sec le filtrat, empâte le résidu à l'éther isopropylique, essore, lave à l'eau, sèche et obtient 2,62 g de produit attendu sous forme de base F = 210¤C. On dissout ce dernier dans un mélange (1-1) de tétrahydrofuranne et d'éthanol, ajoute 0,6 cm3 d'acide chlorhydrique 11,8 N et agite 1 heure à température ambiante. On essore le précipité, lave à l'éthanol, puis à l'éther isopropylique et obtient 2,61 g de produit attendu.

Spectre IR Nujol :

C = O 1 711 $cm^{-1}$

région >C = O } 1 641 $cm^{-1}$
-C = C- }

aromatique 1 602 $cm^{-1}$
1 582 $cm^{-1}$
1 494 $cm^{-1}$

**Exemple 5 : Chlorhydrate de la (16 alpha) (±) 17-benzoyl 14,15-dihydro 14-oxo 17-aza 20,21-dinor-ébunaménine**

**Stade A :** 17-benzoyl 3-16-didehydro 14-15-dihydro 14-oxo 17-aza 20,21-dinor-éburnaménine.

On chauffe à 90¤C pendant 3 heures 35 g de 1-benzoyl 4-[2-(1H-indol 3-yl) éthyl] 3-oxo 2-pipérazine acétate d'éthyle dans 60 cm3 d'oxychlorure de phosphore. On amène à sec, reprend le résidu à l'acétone et verse lentement cette solution sur 70 cm3 d'acide iodhydrique à 56 % dans 500 cm3 d'eau à 0¤C et agite pendant 1 heure, puis essore le précipité. On le dissout à 50¤C dans 250 cm3 d'acétone, ajoute à 50¤C de l'ammoniaque concentrée en 30 minutes et agite pendant 30 minutes à 50¤C. A cette suspension, on ajoute 250 cm3 d'eau et de glace, essore le précipité, lave à l'eau, sèche et obtient 24,7 g de produit attendu F = 236¤C.

**Stade B :** Chlorhydrate de la (16 alpha) (±) 17-benzoyl 14, 15-dihydro 14-oxo 17-aza 20,21-dinor-ébunaménine

On agite 8,3 g de produit obtenu ci-dessus dans 40 cm3 d'acide acétique et ajoute ensuite par petites fractions 4,27 g de borohydrure de sodium en maintenant la température à 25¤C, puis on agite à température ambiante pendant 30 minutes. Après addition de 100 cm3 d'eau glacée, on alcalinise avec de l'ammoniaque concentrée. On essore le précipité, lave à l'eau, sèche et récupère 5,2 g de produit brut. On recristallise 3,5 g de celui-ci dans le diméthylformamide et obtient 2,87 g de produit sous forme de base que l'on dissout dans un mélange (1-1) d'éthanol et de chlorure de méthylène. On ajoute 0,66 cm3 d'acide chlorhydrique 11,8 N et agite pendant 4 heures. On concentre par élimination du chlorure de méthylène, essore le précipité, lave à l'éthanol, sèche et obtient 2,88 g de produit attendu.

Spectre IR ($CHCl_3$) :

C = O 1 708 $cm^{-1}$

C = O }
et C = C } 1 654 $cm^{-1}$

aromatiques { 1 602 $cm^{-1}$
1 580 $cm^{-1}$
1 492 $cm^{-1}$

**Exemple 6 : Dichlorhydrate de la (16alpha) (±) 20,21-dinor 17-aza éburnaménin 14 (15 H)-one**

On porte au reflux 5,2 g de (16 alpha) (±) 17-benzoyl 14,15-dihydro 14-oxo 17-aza 20,21-dinor-éburnaménine dans 80 cm3 d'acide chlorhydrique 5 N pendant 4 heures et demie. On essore le précipité, alcalinise le filtrat par addition d'ammoniaque concentrée et extrait au chlorure de méthylène. On lave à l'eau la phase organique jusqu'à pH neutre, sèche, amène à sec et chromatographie le résidu sur silice (éluant chlorure de méthylène-méthanol 92-8). On isole 2,4 g de produit sous forme de base F = 190¤C. On dissout 1,7 g de base dans 100 cm3 d'un mélange 1/1 acétate d'éthyle-éthanol, ajoute 1,08 cm3 d'acide chlorhydrique 11,8 N et essore le précipité. On obtient 1,86 g de produit attendu.

Spectre IR ($CHCl_3$) :
C = O 1 704 $cm^{-1}$
C = C 1 657 $cm^{-1}$
bande de Bohlmann 2 813 $cm^{-1}$
NH : 3 330 cm-1

**Exemple 7 : Maléate neutre de (16 alpha) (±) 17-méthyl 17-aza 20,21-dinor-éburnaménin 14 (15 H) one**

On dissout 2,85 g de (16 alpha) (±) 20, 21-dinor 17-aza éburnaménin 14 (15H)-one dans 30 cm3 de chlorure de méthylène, ajoute 2,95 g de carbonate de potassium dissous dans 30 cm3 d'eau, puis 300 mg d'hydrogéno sulfate de tétrabutyl ammonium et agite fortement pendant 10 minutes, introduit ensuite 1,3 cm3 d'iodure de méthyle. Après huit heures de forte agitation, on décante la phase organique, lave à l'eau jusqu'à pH neutre sèche, amène à sec et chromatographie le résidu sur silice (éluant chlorure de méthylène-méthanol (92-8)). On recueille 2,24 g de produit sous forme de base F = 148¤C.

On dissout 2,2 g de ce dernier dans un mélange (1/1) acétate d'éthyle-éthanol, ajoute 907 mg d'acide maléique et agite 3 heures. On essore, lave, sèche et obtient 2,5 g de produit attendu F = 240¤C.

**Exemple 8 : Maléate neutre de (16 alpha) (±) 17-(2-phényléthyl) 17-aza 20, 21-dinor-éburnaménin 14 (15 H)-one**

On dissout à 50¤C 2,46 g d'iodure de sodium dans 10 cm3 de diméthylformamide, ajoute 2,2 cm3 de bromure de 2-phényléthyle et maintient sous agitation et à 50¤C pendant 30 minutes. On introduit, dans les mêmes conditions, 2,2 g de (16 alpha) (±) 20, 21-dinor 17-aza-éburnaménin 14-(15H) one, puis 2,27 g de carbonate de potassium et maintient les mêmes conditions pendant 2 heures, puis 72 heures à température ambiante. On essore le précipité, le dissout dans le chlorure de méthylène, sèche la phase organique, amène à sec et empâte le résidu dans 100 cm3 de pentane. On essore et récupère 2,55 g de produit sous forme de base F = 141¤C.

On dissout 2,19 g de base dans 100 cm3 d'un mélange (1/1) d'acétate d'éthyle et d'éthanol, ajoute 684 mg d'acide maléïque dissous dans 10 cm3 d'éthanol. Après 15 heures d'agitation, on essore le précipité et obtient 2,55 g de produit attendu F = 220¤C.

**Exemple 9 : (14alpha,16alpha) (±) 14,15-dihydro 17-aza 20,21-dinor-éburnaménin-14-ol.**

On dissout 7,1 g de la base préparée à l'exemple 6 dans 100 cm3 de tétrahydrofuranne puis ajoute 17 cm3 de diéthyldihydroaluminate de sodium et agite pendant 1 heure à 20¤C. On refroidit à 0¤C, ajoute lentement un mélange tétrahydrofuranne-eau (80-20) puis 100 cm3 d'eau et agite 2 heures en laissant revenir à température ambiante. On extrait au chlorure de méthylène, sèche et concentre à sec sous pression réduite. On reprend le résidu dans 100 cm3 d'acide chlorhydrique N, agite 15 heures à température ambiante, alcalinise à l'aide d'ammoniaque, filtre et extrait au chlorure de méthylène. On filtre le précipité, sèche et concentre le filtrat sous pression réduite et obtient 6,3 g de produit brut que l'on recristallise dans le toluène. F = 246¤C.

| Analyse : $C_{16}H_{19}N_3O$ : 269,346 | | | |
|---|---|---|---|
| Calculé<br>Trouvé | C% 71,35<br>71,6 | H% 7,11<br>7,3 | N% 15,6<br>15,6 |

**Exemple 10 : (E) 2-butènedioate de 16alpha (±) 17-(1-méthyléthyl) 17-aza 20,21-dinoréburnaménin 14(15H)-one.**

On dissout 2 g de la base préparée à l'exemple 6 dans 40 cm3 de tétrahydrofuranne et 10 cm3 d'hexaméthylphisphotriamide, ajoute 5,17 g de carbonate de potassium puis 3,7 cm3 d'iodure d'isopropyle. On agite 72 heures à 50¤C, essore le précipité et concentre à sec le filtrat. On reprend le résidu dans le chlorure de méthylène, lave à l'eau, sèche et élimine le solvant sous pression réduite. Après chromatographie sur silice, (éluant : chlorure de méthylène-méthanol 97-3), en empâte le résidu dans le pentane, sèche à 60¤C sous pression réduite et recueille 2 g de produit attendu sous forme de base que l'on dissout dans l'acétate d'éthyle, ajoute 1,5 g d'acide fumarique dissous dans 30 cm3 d'isopropanol, agite 15 heures à température ambiante, essore le précipité, sèche sous pression réduite à 60¤C et recueille 2,6 g de fumarate neutre attendu. F = 220¤C.

| Analyse : $C_{23}H_{27}N_3O_5$ : 425,488 | | | |
|---|---|---|---|
| Calculé<br>Trouvé | C% 64,93<br>64,8 | H% 6,39<br>6,3 | N% 9,87<br>9,6 |

**Exemple 11 : (Z) 2-butènedioate de (16alpha) (±) 17-éthyl 17-aza 20,21-dinoréburnaménin-14(15H)-one.**

On dissout 0,5 g de la base obtenue à l'exemple 6 dans 10 cm3 de tétrahydrofuranne, ajoute, 0,52 cm3 de triéthylamine puis 0,3 cm3 d'iodure d'éthyle. On chauffe 18 heures au reflux, filtre la suspension et concentre à sec le filtrat sous pression réduite. Après chromatographie sur silice (éluant : chlorure de méthylène-méthanol 95-5), on recueille 0,57 g de produit attendu sous forme de base. F = 120¤C.

On dissout la base dans un mélange acétate d'éthyle-éthanol (1-1), ajoute 0,942 g d'acide maléique, agite 2 heures à température ambiante, filtre et sèche à 60¤C sous pression réduite. On obtient 2,44 g de produit attendu. F = 183¤C.

| Analyse : $C_{18}H_{21}N_3O$, $C_4H_4O_4$ : 411,461 | | | |
|---|---|---|---|
| Calculé<br>Trouvé | C% 64,22<br>64,2 | H% 6,12<br>6,1 | N% 10,21<br>10,1 |

**Exemple 12 : (Z) 2-butènedioate de (16alpha) (±) 17-propyl 17-aza 20,21-dinoréburnaménine 14-(15H)-one.**

On opère comme à l'exemple 11 à partir de 0,2 g de la base préparée à l'exemple 6, 0,208 cm3 de triéthylamine et 0,145 cm3 de n-iodopropyl. On obtient 0,22 g de produit attendu sous forme de base. F = 127¤C.

| Analyse : $C_{19}H_{23}N_3O$, $C_4H_4O_4$ : 425,488 | | | |
|---|---|---|---|
| Calculé<br>Trouvé | C% 64,93<br>65,2 | H% 6,39<br>6,5 | N% 9,87<br>9,8 |

**Exemple 13 : 14alpha, 16alpha (±) 14,15-dihydro 17-méthyl 17-aza 20,21-dinoréburnaménin-14-ol.**

On opère comme à l'exemple 9 en utilisant au départ 2,81 g de la base obtenue à l'exemple 7 et 6 cm3 de diéthyldihydroaluminate de sodium et en opérant cette réduction à -10¤C. On obtient 2,5 g de produit brut que l'on recristallise dans un mélange éthanol-acétate d'ethyle (6-2). On obtient 1,74 g de produit attendu. F = 238¤C.

| Analyse : $C_{17}H_{21}N_3O$ : 283,376 | | | |
|---|---|---|---|
| Calculé<br>Trouvé | C% 72,0<br>72,0 | H% 7,5<br>7,5 | N% 14,5<br>14,7 |

**Exemple 14 : Maléate neutre de (16alpha) (±) (17-méthyl 17-aza 20,21-dinoréburnaménine.**

On dissout 4,2 g de produit préparé comme à l'exemple 13 dans 50 cm3 de toluène, ajoute 80 mg d'acide para-toluènesulfonique et chauffe 5 heures au reflux. On concentre à sec, chromatographie le résidu sur silice (éluant : chlorure de méthylène-méthanol 9-1) et obtient 3,5 g de produit attendu sous forme de base. F = 166¤C.

On dissout 1,5 g de base dans 100 cm3 de mélange acétate d'éthyl-éthanol (1-1), ajoute 0,656 g d'acide maléique, agite 3 heures 45 minutes, essore le précipité, le lave à l'éthanol puis à l'acétate d'éthyle, le sèche à 60¤C sous pression réduite et récupère 1,61 g de maléate attendu. F = 215¤C.

| Analyse : $C_{21}H_{23}N_3O_4$ : 381,435 | | | |
|---|---|---|---|
| Calculé<br>Trouvé | C% 66,13<br>66,0 | H% 6,08<br>5,9 | N% 11,0<br>11,0 |

**Exemple 15 : Maléate neutre de (16alpha) (±) 14,15-dihydro 17-méthyl 17-aza 20,21-dinoréburnaménine.**

On dissout 1,8 g de la base préparée comme à l'exemple 14 dans 100 cm3 d'éthanol, ajoute 90 mg d'oxyde de platine et hydrogène pendant 2 heures et demie. On filtre, concentre à sec le filtrat sous pression réduite et obtient après sèchage à 60¤C, 1,81 g de produit attendu sous forme de base F = 140¤C.

On prépare le maléate comme indiqué à l'exemple 14 à partir de 1,96 g de base et obtient 2,09 g de maléate. F = 212¤C.

| Analyse : $C_{21}H_{25}N_3O_4$ : 383,451 | | | |
|---|---|---|---|
| Calculé<br>Trouvé | C% 65,78<br>65,6 | H% 6,57<br>6,4 | N% 10,96<br>10,8 |

**Exemple 16 : (14alpha, 16alpha) (±) 17-acétyl 14,15-dihydro 14-hydroxy 17-aza 20,21-dinoréburnaménine.**

On refroidit à 10¤C 2,5 g de produit préparé à l'exemple 9 en suspension dans 30 cm3 de tétrahydrofuranne et 2 cm3 de triéthylamine, ajoute lentement 0,66 cm3 de chlorure d'acétyle, agite pendant 1 heure et demie, essore le précipité, le lave à l'eau, le sèche à 80¤C sous pression réduite et obtient 2,6 g de produit attendu. F > 260¤C.

**Example 17 : (16alpha) (±) 17-acétyl 17-aza 20,21-dinoréburnaménine.**

On ajoute 52 mg d'acide para-toluènesulfonique dans une suspension de 2,6 g de produit préparé à l'exemple 16 dans 80 cm3 de toluène et chauffe 6 heures au reflux. On concentre à sec sous pression

réduite la solution obtenue, lave à l'eau le résidu, le sèche à 80¤C et obtient 2,1 g de produit attendu. F = 180¤C.

**Exemple 18 : Maléate neutre de (16alpha) (±) 17-aza 20,21-dinoréburnaménine.**

On chauffe 5 heures au reflux 2,1 g de produit préparé à l'exemple 17 dans un mélange comprenant 25 cm3 d'éthanol et 2 cm3 de potasse. On ajoute 50 cm3 d'eau, extrait au chlorure de méthylène, lave à l'eau, sèche, concentre à sec sous pression réduite, chromatographie le résidu sur silice (éluant : chlorure de méthylène-méthanol 9-1) et obtient 1,42 g de produit attendu. F = 135¤C.

On prépare le maléate comme indiqué à l'exemple 14 à partir de 1,4 g de base et obtient 1,8 g de maléate attendu. F = 246¤C.

| Analyse : $C_{20}H_{21}N_3O_4$ : 367,408 | | | |
|---|---|---|---|
| Calculé<br>Trouvé | C% 65,38<br>65,2 | H% 5,76<br>5,7 | N% 11,44<br>11,4 |

**Exemple 19 : (14béta, 16alpha) (±) 14,15-dihydro 17-(1-méthyléthyl) 17-aza 20,21-dinoréburnaménin-14-ol.**

On opère comme à l'exemple 9 en utilisant au départ 4,5 g de la base préparée à l'exemple 10 et 9,4 cm3 de diéthyldihydroaluminate de sodium et en opérant cette réduction à -5¤C. On obtient 3,7 g de produit attendu. F = 211¤C.

**Exemple 20 : (16alpha) (±) 17-(1-méthyléthyl) 17-aza 20,21-dinoréburnaménine.**

On opère comme à l'exemple 17 en utilisant au départ 3,2 g de produit préparé à l'exemple19 et obtient 2,8 g de produit attendu. F < 50¤C. **Exemple 21: Fumarate de (16alpha) (±) 14,15-dihydro 17-(1-méthyléthyl) 17-aza 20,21-dinoréburnaménine.**

On opère comme à l'exemple 15 à partir de 2,8 g de produit obtenu à l'exemple 20 et obtient 2,77 g de produit attendu sous forme de base, F = 130¤C, puis 3,28 g de fumarate, F = 230¤C.

| Analyse : $C_{25}H_{31}N_3O_6$ : 469,535 | | | |
|---|---|---|---|
| Calculé<br>Trouvé | C% 63,95<br>63,6 | H% 6,65<br>6,7 | N% 8,95<br>8,7 |

**Exemple 22 : (16alpha) 17-benzoyl 14,15-dihydro 11-méthyl 14-oxo 17-aza 20,21-dinoréburnaménine.**

**Stade A :** 2-[[2-(6-méthyl 1H-indol-3-yl) éthyl] amino] acétonitrile.

On dissout à 40¤C sous atmosphère inerte 17 g de 6-méthyl tryptamine dans 500 cm3 de tétrahydrofuranne, laisse revenir à température ambiante, ajoute 14,6 cm3 de 1,5-diaza [5.4.0] undec-5-ène; puis 6,2 cm3 de chloroacétonitrile et agite 15 heures. On élimine le solvant sous pression réduite, reprend le résidu dans 250 cm3 de chlorure de méthylène, lave à l'eau, sèche et concentre à sec. On obtient 25 g de produit brut que l'on purifie par chromatographie sur silice (éluant : chlorure de méthylèneacétate d'éthyl 3-7). On recueille 13,2 g de produit attendu. F = 92¤C.

**Stade B :** Chlorhydrate de 1-[2-(6-méthyl 1H-indol-3-yl) éthyl] éthanediamine.

On opère comme au stade B de l'exemple 1 à partir de 13,2 g de produit obtenu au stade A précédent et obtient 13,4 g de base. On dissout 0,170 g de base dans 1 cm3 d'isopropanol, ajoute à 0¤C, 0,13 cm3 d'acide chlorhydrique concentré, agite 20 minutes, filtre le précipité, le lave à l'éther isopropylique, le sèche et obtient 0,170 g du chlorhydrate attendu. F > 260¤C.

**Stade C :** 4-[2-(6-méthyl 1H-indol-3-yl) éthyl] 3-oxo 2-pipérazine acétate d'éthyle.

On opère comme au stade C de l'exemple 1 en utilisant 13 g de la base préparée comme au stade B précédent et 16,4 cm3 de maléate d'éthyle. On obtient 10 g de produit attendu.

**Stade D :** 1-benzoyl 4-[2-(6-méthyl 1H-indol-3-yl) éthyl] 3-oxo 2-pipérazine acétate d'éthyle.

On opère comme au stade D de l'exemple 1 en utilisant 10 g du produit préparé au stade C précédent et 3,36 cm3 de chlorure de benzoyle. On obtient 13 g de produit attendu. F = 155¤C.

**Stade E :** 17-benzoyl 3,16-didehydro 14,15-dihydro 11-méthyl 14-oxo 17-aza 20,21-dinoréburnaménine.

On chauffe 3 heures à 75¤C 11,6 g de produit obtenu au stade D précédent dans 23,5 cm3 d'oxychlorure de phosphore. On laisse revenir à température ambiante, verse sur une solution aqueuse glacée d'acide perchlorique. On agite 1 heure à 0¤C, filtre le précipité, le reprend à l'acétone et chauffe à 50¤C. On ajoute en 30 minutes 120 cm3 d'ammoniaque concentrée, maintient sous agitation 1 heure 15 minutes à 50¤C, laisse revenir à température ambiante, verse dans l'eau glacée, filtre le précipité, le lave à l'eau et le sèche à 50¤C sous pression réduite. On obtient 8,2 g de produit attendu. F = 250¤C.

**Stade F :** (16alpha) 17-benzoyl 14,15-dihydro 11-méthyl 14-oxo 17-aza 20,21-dinoréburnaménine.

On agite 8,2 g de produit obtenu au stade E précédent dans 85 cm3 d'acide acétique et ajoute par petites fractions 4,6 g de borohydrure de sodium en maintenant la température inférieure à 50¤C. On agite 1 heure à température ambiante, élimine l'acide acétique, alcalinise avec de l'ammoniaque concentrée, agite 15 minutes, filtre le précipité, le lave à l'eau, le sèche à 70¤C et récupère 5,93 g de produit attendu. F = 262¤C.

Spectre IR ($CHCl_3$)

| | | |
|---|---|---|
| -C=O | : | 1 717 $cm^{-1}$, 1 700 $cm^{-1}$ |
| C=C | : | 1 653 $cm^{-1}$ |
| système conjugué + aromatique | } | 1 638 $cm^{-1}$, 1 616 $cm^{-1}$, 1 576 $cm^{-1}$ |

**Exemple 23 : (16alpha) (±) 11-méthyl 17-aza 20,21-dinoréburnaménin 14(15H)-one.**

On chauffe 6 heures au reflux 5,9 g de produit préparé à l'exemple 22 dans 72 cm3 d'acide chlorhydrique 5N. On élimine le précipité par filtration, alcalinise le filtrat avec une solution aqueuse d'hydroxyde de sodium, extrait au chlorure de méthylène, lave la phase organique à l'eau, la sèche et la concentre à sec sous pression réduite. On chromatographie le résidu sur silice (éluant : chlorure de méthylène-méthanol 96-4) et obtient 3,1 g de produit attendu. F = 196¤C.

Spectre IR ($CHCl_3$)

| | | |
|---|---|---|
| NH | : | 3 470 $cm^{-1}$ |
| -C=O | : | 1 702 $cm^{-1}$ |
| -C=C- | : | 1 659 $cm^{-1}$ |
| aromatique | : | 1 612 $cm^{-1}$, 1 600 $cm^{-1}$ |

**Exemple 24 : Maléate neutre de (16alpha) (±) 11,17-diméthyl 17-aza 20,21-dinoréburnaménin 14-(15H)-one.**

On opère comme à l'exemple 7 à partir de 3,1 g de produit préparé à l'exemple 23, 3,05 g de carbonate de potassium, 1,4 cm3 d'iodure de méthyle et 0,3 g d'hydrogénosulfate de tétrabutylammonium. On obtient 2,1 g de produit attendu sous forme de base F = 171¤C, puis 2,07 g de maléate attendu F = 215¤C.

| Analyse: $C_{18}H_{21}N_3O$, $C_4,H_4O_4$ : 411,467 | | | |
|---|---|---|---|
| Calculé<br>Trouvé | C% 64,22<br>64,2 | H% 6,12<br>6 | N% 10,21<br>10,1 |

**Exemple 25 : (16alpha) (±) 14,15-dihydro 11-méthoxy 14-oxo 17-aza 20,21-dinoréburnaménine.**

**Stade A :** 2-[[2-(6-méthoxy 1H-indol-3-yl) éthyl] amino] acétonitrile.

On chauffe au reflux 28,5 g de 6-méthoxy tryptamine dans 300 cm3 de tétrahydrofuranne en présence de 42 cm3 de triéthylamine. On ajoute en 2 heures 11,4 cm3 de chloroacétonitrile, poursuit le chauffage pendant 1 heure, laisse revenir à température ambiante, élimine par filtration le précipité, concentre à sec le filtrat sous pression réduite, reprend le résidu dans l'acétate d'éthyle, lave à l'eau, sèche et élimine le solvant sous pression réduite. On récupère 17,9 g de produit attendu. F ≃ 80¤C.

**Stade B :** Chlorhydrate de 1-[2-(6-méthoxy 1H-indol-3-yl) éthyl] éthane diamine.

On opère comme au stade B de l'exemple 1 à partir de 17,9 g du produit obtenu an stade A précédent et obtient 17,2 g de produit attendu. On dissout 0,58 g de base dans 20 cm3 d'acétate d'éthyle et 30 cm3 d'éthanol, ajoute à 0¤C, 0,43 cm3 d'acide chlorhydrique, agite 1 heure à 20¤C, essore le précipité, le sèche à 20¤C sous pression réduite et obtient 0,440 g de chlorhydrate attendu. F = 200¤C.

**Stade C :** 4-[2-(6-méthoxy 1H-indol-3-yl) éthyl] 3-oxo 2-pipérazine acétate d'éthyle.

On opère comme au stade C de l'exemple 1 en utilisant 17,2 g de la base préparée au stade B précédent et 17,9 cm3 de maléate de diéthyle. On obtient 14 g de produit attendu.

**Stade D :** 1-benzoyl 4-[2-(6-méthoxy 1H-indol-3-yl) éthyl] 3-oxo 2-pipérazine acétate d'éthyle.

On opère comme au stade D de l'exemple 1 en utilisant 14 g du produit préparé au stade C précédent, 8,2 cm3 de triéthylamine et 4,5 cm3 de chlorure de benzoyle. On obtient 18 g de produit attendu.

**Stade E :** 17-benzoyl 3,16-didéhydro 14,15-dihydro 11-méthoxy 14-oxo 17-aza 20,21-dinoréburnaménine.

On opère comme au stade E de l'exemple 22 en utilisant 18 g de produit préparé au stade D précédent, 36 cm3 d'oxychlorure de phosphore, 18 cm3 d'acide perchlorique et 180 cm3 d'ammoniaque concentrée. On obtient 12 g de produit attendu. F = 220¤C.

**Stade F :** (16alpha) (±) 14,15-dihydro 11-méthoxy 14-oxo 17-aza 20,21-dinoréburnaménine.

On opère comme au stade F de l'exemple 22 en utilisant 12 g de produit préparé au stade E précédent, 5,8 g de borohydrure de sodium et 300 cm3 d'acide acétique. On obtient 10 g de produit attendu. F > 260¤C.

Spectre IR ($CHCl_3$)

| | |
|---|---|
| >C=O | : 1 705 $cm^{-1}$, 1 650 $cm^{-1}$ |
| C=C + aromatique | 1 656 $cm^{-1}$, 1 615 $cm^{-1}$, 1 602 $cm^{-1}$, 1 580 $cm^{-1}$, 1 488 $cm^{-1}$ |

**Exemple 26 : (16alpha) (±) 11-méthoxy 17-aza 20,21-dinoréburnaménin 14(15H)-one.**

On opère comme à l'exemple 23 en utilisant 10 g de produit préparé comme à l'exemple 25 et 200 cm3 d'acide chlorhydrique 5N. On obtient 4,64 g de produit attendu. F = 142¤C.

Spectre IR ($CHCl_3$)

| | |
|---|---|
| -C=O | : 1 704 $cm^{-1}$ |
| C=C- + aromatique | 1 661 $cm^{-1}$, 1 615 $cm^{-1}$, 1 578 $cm^{-1}$, 1 487 $cm^{-1}$ |
| NH | : 3 330 $cm^{-1}$ |

**Exemple 27 : Maléate neutre de la (16alpha) (±) 11-méthoxy 17-méthyl 17-aza 20,21-dinoréburnaménin 14(15H)-one.**

On opère comme à l'exemple 7 en utilisant 2,5 g de produit préparé à l'exemple 26, 2,32 g de carbonate de potassium, 1,05 cm3 d'iodure de méthyle et 0,25 g d'hydrogénosulfate de tétrabutylammonium. On obtient 1,5 g de produit attendu sous forme de base F = 143¤C, puis 1,67 g de maléate attendu F = 210¤C.

| Analyse : $C_{22}H_{25}N_3O_6$ : 427,46 | | | |
|---|---|---|---|
| Calculé | C% 61,82 | H% 5,89 | N% 9,83 |
| Trouvé | 61,6 | 5,9 | 9,8 |

**Exemple 28 : (16alpha) (±) 17-benzoyl 14,15-dihydro 10-méthoxy 14-oxo 17-aza 20,21-dinoréburnaménine.**

**Stade A :** 2-[[2-(5-méthoxy 1H-indol-3-yl) éthyl] amino] acétonitrile.

On opère comme au stade A de l'exemple 25 en utilisant 25 g de 5-méthoxy tryptamaine, 10 cm3 de chloroacétonitrile et 37 cm3 de triéthylamine. On obtient 20,9 g de produit attendu.

**Stade B :** 1-[2-(5-méthoxy 1H-indol-3-yl) éthyl] éthane diamine.

On opère comme au stade B de l'exemple 1 en utilisant au départ 2 g de produit préparé au stade A précédent et obtient 2,02 g de produit attendu.

**Stade C :** 4-[2-(5-méthoxy 1H-indol-3-yl) éthyl] 3-oxo 2-pipérazine acétate d'éthyle.

On opère comme au stade C de l'exemple 1 en utilisant au départ 21,4 g de produit préparé comme au stade B précédent et 22,3 cm3 de maléate de diéthyle. On obtient 13,9 g de produit attendu.

**Stade D :** 1-benzoyl 4-[2-(5-méthoxy 1H-indol-3-yl) éthyl] 3-oxo 2-pipérazine acétate d'éthyle.

On opère comme au stade D de l'exemple 1 en utilisant 13,9 g du produit préparé au stade C, 8,2 cm3 de triéthylamine et 4,5 cm3 de chlorure de benzoyle. On obtient 18 g de produit attendu.

**Stade E :** 17-benzoyl 3,16-didehydro 14,15-dihydro 10-méthoxy 14-oxo 17-aza 20,21-dinoréburnaménine.

On opère comme au stade E de l'exemple 22 en utilisant 18 g de produit préparé au stade D précédent, 36 cm3 d'oxychlorure de phosphore, 36 cm3 d'acide perchlorique et 180 cm3 d'ammoniaque concentrée. On obtient 11 g de produit attendu. F = 243¤C.

**Stade F :** (16alpha) (±) 17-benzoyl 14,15-dihydro 10-méthoxy 14-oxo 17-aza 20,21-dinoréburnaménine.

On opère comme au stade F de l'exemple 22 en utilisant au départ 11 g de produit obtenu au stade E précédent, 5,3 g de borohydrure de sodium et 250 cm3 d'acide acétique. On obtient 8,7 g de produit attendu. F ≃ 260¤C.

Spectre IR (nujol)

-C=O : 1 699 $cm^{-1}$

-C(=O)-C=C-N< doublet ≃ 1 650 $cm^{-1}$

aromatique : 1 609 $cm^{-1}$, 1 600 $cm^{-1}$, 1 580 $cm^{-1}$

**Exemple 29 : (16alpha) (±) 10-méthoxy 17-aza 20,21-dinoréburnaménin 14(15H)-one.**

On opère comme à l'exemple 23 en utilisant 7,1 g de produit préparé à l'exemple 28 et 250 cm3 d'acide chlorhydrique 5N. On obtient 3,74 g de produit attendu. F = 209¤C.

Spectre IR ($CHCl_3$)

-C=O : 1 701 $cm^{-1}$

-C=C- + aromatique } 1 656 $cm^{-1}$, 1 624 $cm^{-1}$, 1 614 $cm^{-1}$, 1 580 $cm^{-1}$

NH : 3 320 $cm^{-1}$

**Exemple 30 : Maléate neutre de la (16alpha) (±) 10-méthoxy 17-méthyl 17-aza 20,21-dinoréburnaménine 14(15H)-one.**

On dissout 2 g du produit obtenu à l'exemple 29 dans un mélange comprenant 3,8 cm3 de triéthylamine et 100 cm3 de tétrahydrofuranne et ajoute à 60¤C en 4 heures, 1,7 cm3 d'iodure de méthyle. On chauffe 30 minutes au reflux, laisse revenir à température ambiante, filtre le précipité, concentre à sec le filtrat, purifie le résidu par chromatographie sur silice (éluant : chlorure de méthylène-méthanol 95-5) et récupère après séchage à 50¤C sous pression réduite 1,4 g de produit attendu. F = 173¤C. On dissout 1,86 g de base préparée comme ci-dessus dans 100 cm3 d'acétate d'éthyle, ajoute 0,693 g d'acide maléique dissous dans 100 cm3 d'acétate d'éthyle bouillant et agite pendant 5 heures à température ambiante. On essore, lave à l'acétate d'éthyle, sèche à 70¤C sous pression réduite et obtient 2,34 g de maléate neutre attendu. F = 192¤C.

| Analyse : $C_{18}H_{21}N_3O_2,C_4H_4O_4$ : 427,46 | | | |
|---|---|---|---|
| Calculé | C% 61,82 | H% 5,89 | N% 9,83 |
| Trouvé | 61,5 | 5,9 | 9,6 |

**Exemple 31 : 17-dodécanoyl 3,16-didéhydro 14,15-dihydro 14-oxo 17-aza 20,21-dinoréburnaménine.**

**Stade A :** 4-[2-(1H-indol-3-yl) éthyl] 3-oxo 1-(1-oxodocécyl) 2-pipérazine acétate d'éthyle.

On dissout 2,14 g de produit obtenu comme au stade C de l'exemple 1 dans un mélange comprenant 1,4 cm3 de triéthylamine et 25 cm3 de tétrahydrofuranne et ajoute, goutte à goutte, 1,5 cm3 de chlorure de lauroyle. On agite 30 minutes, filtre le précipité, concentre à sec le filtrat, reprend le résidu dans le chlorure de méthylène, lave à l'eau, sèche et élimine le solvant sous pression réduite à 50¤C. On obtient 3,2 g de produit attendu.

**Stade B :** 17-dodécanoyl 3,16-didéhydro 14,15-dihydro 14-oxo 17-aza 20,21-dinoréburnaménine.

On dissout 6,4 g de produit préparé comme au stade A dans 25 cm3 d'oxychlorure de phosphore et chauffe 1 heure à 90¤C. Onconcentre à sec, reprend le résidu dans 50 cm3 d'acétone, chauffe à 55¤C, ajoute en 30 minutes 60 cm3 d'ammoniaque concentrée, agite 1 heure, verse dans l'eau glacée, agite 1 heure à 0¤C, essore le précipité, le lave à l'eau, l'empâte dans l'acétone, le sèche à 50¤C sous pression réduite et obtient 1,9 g de produit brut que l'on reprend dans le chlorure de méthylène ; on filtre la solution sur silice, concentre à sec le filtrat, reprend le résidu dans du méthanol, sèche sous pression réduite à 50¤C et recueille 1,2 g de produit attendu. F = 146¤C.

| Analyse: $C_{28}H_{37}N_3O_2$ : 447,626 | | | |
|---|---|---|---|
| Calculé | C% 75,13 | H% 8,33 | N% 9,39 |
| Trouvé | 75 | 8,4 | 9,2 |

**Exemple 32 : (16alpha) (±) 17-benzoyl 14,15-dihydro 10-chloro 14-oxo 17-aza 20,21-dinoréburnaménine.**

**Stade A :** 2-[[2-(5-chloro 1H-indol-3-yl) éthyl] amino] acétonitrile.

On opère comme au stade A de l'exemple 22 en utilisant au départ 21,4 g de 5-chloro tryptamine, 7,7 cm3 de chloroacétonitrile, 18,1 cm3 de 1,5-diaza [5.4.0.] undec-5-ène dans 210 cm3 de tétrahydrofuranne et obtient 19,3 g de produit attendu. F = 80¤C.

**Stade B :** Dichlorhydrate de la 1-[2-(5-chloro 1H-indol-3-yl) éthyl] éthane diamine.

On opère comme au stade B de l'exemple 1 àpartir de 0,5 g de produit obtenu au stade A précédent. On obtient 0,43 g de produit sous forme de base que l'on transforme en dichlorhydrate par acion de 0,3 cm3 d'acide chlorhydrique 11,8N. On obtient 0,400 g de produit attendu. F = 220¤C.

**Stade C :** 4-[2-(5-chloro 1H-indol-3-yl) éthyl] 3-oxo 2-pipérazine acétate d'éthyle.

On opère comme au stade C de l'exemple 1 en utilisant 18,8 g de la base préparée comme au stade B précédent et 19 cm3 de maléate de diéthyle. On obtient 11,03 g de produit attendu. F = 136¤C.

**Stade D :** 1-benzoyl 4-[2-(5-chloro 1H-indol-3-yl) éthyl] 3-oxo 2-pipérazine acétate d'éthyle.

On opère comme au stade D de l'exemple 1 en utilisant 11 g du produit obtenu au stade C précédent et 3,5 cm3 de chlorure de benzoyle. On obtient 13 g de produit attendu.

**Stade E :** 17-benzoyl 10-chloro 3,16-didehydro 14,15-dihydro 14-oxo 17-aza 20,21-dinoréburnaménine.

On opère comme au stade E de l'exemple 22 en utilisant 7,1 g de produit préparé au stade D précédent, 11 cm3 d'oxychlorure de phosphore, 14 cm3 d'acide perchlorique et 70 cm3 d'ammoniaque concentrée. On obtient le produit brut attendu que l'on utilise tel quel pour le stade suivant.

**Stade F :** (16alpha) (±) 17-benzoyl 14,15-dihydro 10-chloro 14-oxo 17-aza 20,21-dinoréburnaménine.

On opère comme au stade F de l'exemple 22 à partir du produit obtenu au stade E précédent, 2,88 g de borohydrure de sodium et 150 cm3 d'acide acétique. On obtient 0,8 g de produit attendu. F > 260¤C.

| Spectre IR ($CHCl_3$) | |
|---|---|
| -C=O<br>aromatique | 1 711 $cm^{-1}$, 1 652 $cm^{-1}$<br>1 602 $cm^{-1}$, 1 575 $cm^{-1}$, 1 569 $cm^{-1}$ |

**Exemple 33 : (16alpha) (±) 10-chloro 17-aza 20,21-dinoréburnaménin 14(15H)-one.**

On opère comme àl'exemple 23 à partir de 0,8 g de produit préparé à l'exemple 32 et 50 cm3 d'acide chlorhydrique 5N. On obtient 0,5 g de produit attendu. F = 200¤C.

Spectre IR ($CHCl_3$)

-C=O : 1 710 $cm^{-1}$

C=C C=N + aromatique } 1 657 $cm^{-1}$, 1 602 $cm^{-1}$, 1 574 $cm^{-1}$, 1 565 $cm^{-1}$

**Exemple 34 : Maléate neutre de la (16alpha) (±) 10-chloro 17-méthyl 17-aza 20,21-dinoréburnaménin 14(15H-one.**

On opère comme à l'exemple 7 à partir de 0,5 g du produit obtenu à l'exemple 33, 0,45 g de carbonate de potassium, 0,2 cm3 d'iodure de méthyle et 0,05 g d'hydrogénosulfate de tétrabutylammonium. On obtient 0,24 g de produit attendu sous forme de base F = 211¤C, puis 0,195 g de maléate attendu F = 214¤C.

| Analyse : $C_{21}H_{22}N_3ClO_5$ : 431,879 | | | | |
|---|---|---|---|---|
| Calculé<br>Trouvé | C% 58,4<br>58,4 | H% 5,13<br>5,1 | N% 9,73<br>9,7 | Cl% 8,21<br>8,1 |

**Exemple 35 :**

On a préparé des comprimés répondant à la formule suivante :

| | |
|---|---|
| - produit de l'exemple 7<br>- excipient qsp | 50 mg,<br>350 mg |

(détail de l'excipient : lactose, talc, amidon, stéarate de magnésium).

**Activité analgésique :** - Test de la plaque chaude :

Des souris femelles pesant 22 à 24 g sont placées une par une sur une plaque de cuivre maintenue à 56¤C : la réaction à la douleur se manifeste par le léchage des pattes avant de l'animal ; le temps de cette réaction est noté et l'on ne retient que les souris réagissant en moins de 8 secondes.

Les animaux sont répartis par groupes homogènes et traités par le produit à étudier administré par voie buccale, un groupe ne recevant que le véhicule. Le temps de réaction à la douleur est de nouveau mesuré 30 à 60 minutes après le traitement. La dose active ou $DA_{100}$ est la dose qui augmente le temps de réaction de 100 %, 30 minutes après le traitement, compte tenu des variations du temps de réaction des animaux témoins.

**Résultats :**

| Produit de l'exemple | $DA_{100}$ en mg/kg |
|---|---|
| 6 | 100 |
| 7 | 100 |
| 12 | ≤ 100 |
| 15 | 75 |
| 18 | 100 |

**Revendications**
**Revendications pour les Etats contractants suivants : AT, BE, DE, FR, GB, IT, LI, LU, NL, SE**

**1.** Composés de formule (I) :

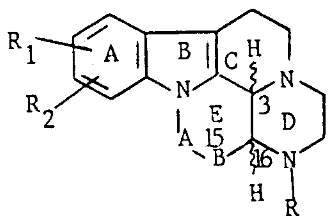

(I)

dans laquelle $R_1$ et $R_2$, identiques ou différents, représentent un atome d'hydrogène, un atome d'halogène, un radical alcoyle renfermant de 1 à 5 atomes de carbone, alcoxy renfermant de 1 à 5 atomes de carbone, un radical hydroxy, trifluorométhyle ou nitro, R représente un atome d'hydrogène, un radical alcoyle renfermant de 1 à 5 atomes de carbone, un radical arylalcoyle renfermant de 7 à 12 atomes de carbone, un radical benzoyle ou alcoylcarbonyle dans lequel le radical alcoyle renferme de

1 à 12 atomes de carbone, et dans laquelle le groupement :

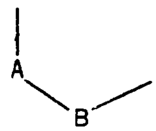

représente soit

i) 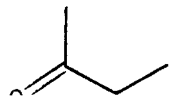

soit

ii) 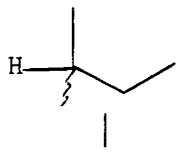 ,

soit

iii) 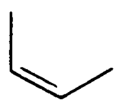 ,

soit

iiii) 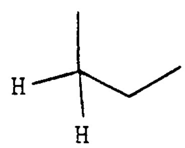

lesdits produits de formule (I) étant sous toutes les formes isomères possibles racémiques ou optiquement actives, ainsi que les sels d'addition avec les acides minéraux ou organiques desdits produits de formule (I).

**2.** Composés de formule (I), tels que définis à la revendication 1, caractérisés en ce que que $R_1$ et $R_2$ représentent un atome d'hydrogène, un radical méthyle, éthyle, méthoxy ou éthoxy, un atome de chlore, un radical hydroxy, trifluorométhyle ou nitro, et R représente un atome d'hydrogène, un radical méthyle, éthyle ou propyle, benzyle, phénéthyle, ou benzoyle, sous toutes les formes isomères possibles racémiques ou optiquement actives, ainsi que les sels d'addition avec les acides minéraux ou organiques.

**3.** Composés de formule (I), tels que définis aux revendications 1 et 2, caractérisés en ce que R2 représente un atome d'hydrogène, R1 représente un atome d'hydrogène, un radical méthoxy ou éthoxy, un atome de chlore sous toutes les formes isomères possibles racémiques ou optiquement actives, ainsi que les sels d'addition avec les acides minéraux ou organiques.

**4.** Composés de formule (I), tels que définis aux revendications 1 à 3, caractérisés en ce que l'atome d'hydrogène en position 3 et l'atome d'hydrogène en position 16 sonttrans sous toutes les formes isomères possibles racémiques ou optiquement actives ainsi que les sels d'addition avec les acides

minéraux ou organiques.

5.
- La (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine, et leurs sels d'addition avec les acides.

6. Procédé de préparation des composés de formule (I), telle que définie à la revendication 1, caractérisé en ce que l'on fait réagir un composé de formule (II) :

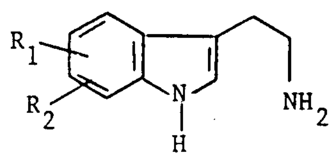

(II)

dans laquelle $R_1$ et $R_2$ ont les significations données ci-dessus avec le chloroacétonitrile de manière à obtenir un composé de formule (III) :

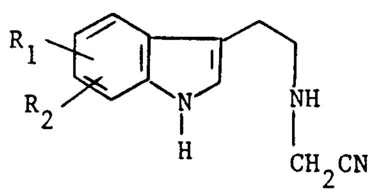

(III)

que l'on réduit en un composé de formule (IV) :

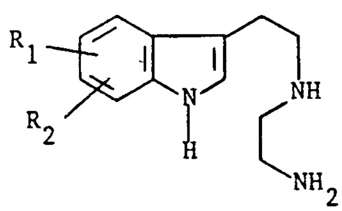

(IV)

que l'on condense avec un maléate ou un fumarate d'alcoyle de formule

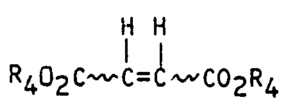

dans laquelle $R_4$ est un radical alcoyle renfermant de 1 à 4 atomes de carbone pour obtenir un composé de formule (V) :

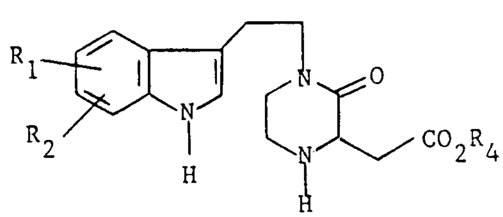

(V)

sur lequel on fait réagir un réactif susceptible d'introduire un radical R', R' ayant les valeurs indiquées pour R à l'exception de la valeur hydrogène, pour obtenir un composé de formule (VI) :

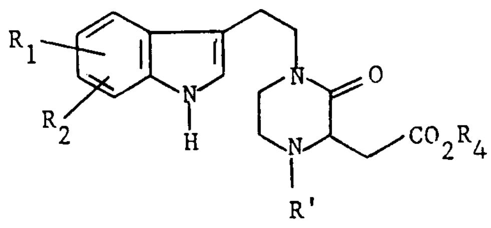

(VI)

sur lequel on fait réagir un agent de cyclisation pour obtenir un composé de formule (VII) :

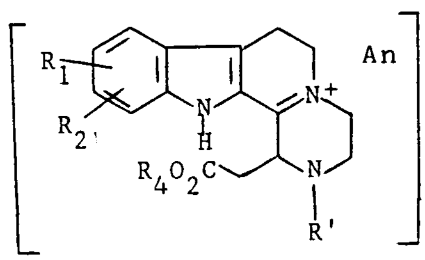

(VII)

dans lequel $R_1$, $R_2$, $R_4$ et R' ont les significations indiquées précédemment et An représente un anion dérivé d'un acide fort et que, soit l'on réduit le composé de formule (VII) en composé de formule (VIII) dans laquelle les deux atomes d'hydrogène en position 3 et 16 sont en position cis :

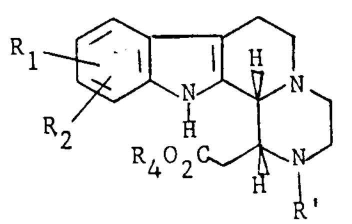

(VIII)

dont on élimine le cas échéant le groupement R' dans le cas où celui-ci représente un radical benzoyle ou alcoylcarbonyle et que l'on cyclise pour obtenir un composé de formule

$(I_{A_{1b}})$

correspondant à un produit de formule (I) dans laquelle :

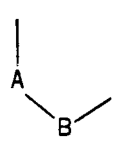

représente

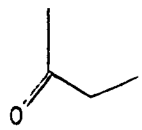

les deux atomes d'hydrogène en position 3 et 16 sont en position cis et R représente un atome d'hydrogène et composé de formule

$(I_{A_{1b}})$ ,

que l'on soumet le cas échéant à l'action d'un réactif susceptible d'introduire un radical R', R' ayant toutes les valeurs indiquées pour R à l'exception de la valeur hydrogène, pour obtenir un composé de formule

$(I_{A_{1c}})$

dans laquelle R a la signification indiquée ci-dessus pour R', soit l'on cyclise le composé de formule (VII) en composé de formule (IX) :

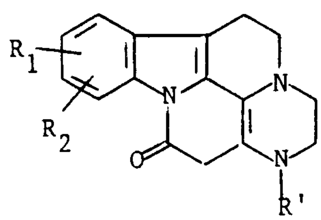

(IX)

que l'on réduit pour obtenir un composé de formule

$(I_{A_{2a}})$

correspondant à un produit de formule (I) dans laquelle :

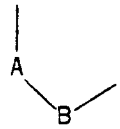

représente

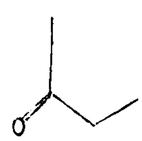

les deux atomes d'hydrogène en position 3 et 16 sont en position trans et R a la signification indiquée ci-dessus pour R', dont on élimine le cas échéant, le groupement R' dans le cas où celui-ci représente un radical benzoyle ou alcoylcarbonyle, pour obtenir un composé de formule

$(I_{A_{2b}})$

dans laquelle R est un atome d'hydrogène, que l'on soumet, le cas échéant, à l'action d'un réactif susceptible d'introduire un radical R', R' ayant les valeurs indiquées pour R à l'exception de la valeur hydrogène pour obtenir un composé de formule

$(I_{A_{2c}})$

dans laquelle R a la signification indiquée ci-dessus pour R', lesdits composés de formules

$(I_{A_{1b}})$

$(I_{A_{1c}})$, $(I_{A_{2a}})$, $(I_{A_{2b}})$ et $(I_{A_{2c}})$

étant, si désiré, réduits en composés correspondants de formules

$(I_{B_{1b}})$, $(I_{B_{1c}})$, $(I_{B_{2a}})$,

$(I_{B_{2b}})$ et $(I_{B_{2c}})$

représentant les composés de formule (I) dans laquelle :

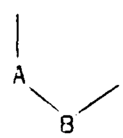

représente

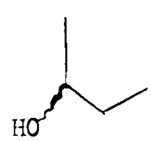

lesdits composés de formules

$(I_{B_{1b}})$, $(I_{B_{1c}})$, $(I_{B_{2a}})$,

$(I_{B_{2b}})$, $(I_{B_{2c}})$

étant, si désiré, soumis à l'action d'un agent déshydratant pour obtenir les composés correspondants de formules

$(I_{C_{1b}})$, $(I_{C_{1c}})$, $(I_{C_{2a}})$, $(I_{C_{2b}})$ et $(I_{C_{2c}})$

représentant les composés de formule (I) dans laquelle:

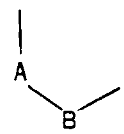

représente

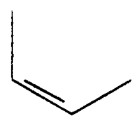

lesdits composés de formules

$(I_{C_{1b}})$, $(I_{C_{1c}})$, $(I_{C_{2a}})$,

$(I_{C_{2b}})$ et $(I_{C_{2c}})$

étant, si désiré, soumis à l'action d'un agent de réduction, pour obtenir les composés correspondants de formules

$(I_{D_{1b}})$,

$(I_{D_{1c}})$, $(I_{D_{2a}})$, $(I_{D_{2b}})$ et $(I_{D_{2c}})$

eprésentant les composés de formule (I) dans laquelle :

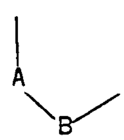

représente

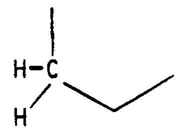

et traite si désiré tous les produits de formule(I) obtenus par un acide minéral ou organique pour en former les sels.

**7.** A titre de médicaments, les composés tels que définis par la formule (I) de la revendication 1, lesdits produits de formule (I) étant sous toutes les formes isomères possibles racémiques ou optiquement actives, ainsi que les sels d'addition avec les acides minéraux ou organiques pharmaceutiquement acceptables.

**8.** A titre de médicaments, les composés tels que définis aux revendications 2 à 4.

**9.** A titre de médicaments, les composés de la revendication 5.

**10.** Les compositions pharmaceutiques contenant à titre de principe actif, l'un au moins des médicaments tels que définis à l'une quelconque des revendications 7 à 9.

**11.** A titre de produits industriels, les composés de formules (III), (IV') correspondant à la formule (IV) dans laquelle lorsque l'un de $R_1$ ou $R_2$ représente un atome d'hydrogène, l'autre de $R_1$ ou $R_2$ ne représente pas un radical méthoxy, les composés (V), (VI), (VII), (VIII) et (IX).

**Revendications pour l'Etats contractant suivant : ES**

**1.** Procédé pour préparer les composés de formule (I) :

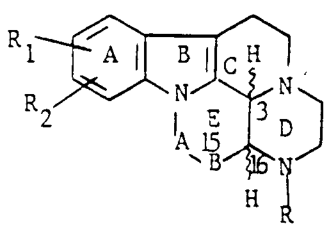

(I)

dans laquelle $R_1$ et $R_2$, identiques ou différents, représentent un atome d'hydrogène, un atome d'halogène, un radical alcoyle renfermant de 1 à 5 atomes de carbone, alcoxy renfermant de 1 à 5 atomes de carbone, un radical hydroxy, trifluorométhyle ou nitro, R représente un atome d'hydrogène, un radical alcoyle renfermant de 1 à 5 atomes de carbone, un radical arylalcoyle renfermant de 7 à 12 atomes de carbone, un radical benzoyle ou alcoylcarbonyle dans lequel le radical alcoyle renferme de 1 à 12 atomes de carbone, et dans laquelle le groupement :

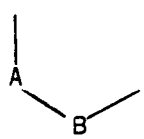

représente soit

i) 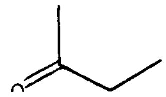

soit

ii) 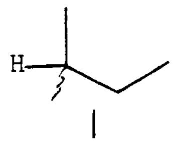 ,

soit

iii) 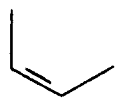 ,

soit

iiii) 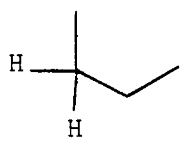

lesdits produits de formule (I) étant sous toutes las formes isomères possibles racémiques ou optiquement actives, ainsi que les sels d'addition avec les acides minéraux ou organiques desdits produits de formule (I), caractérisé en ce que l'on fait réagir un composé de formule (II) :

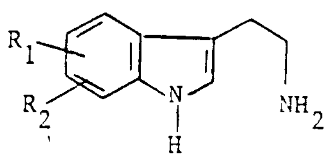

(II)

dans laquelle $R_1$ et $R_2$ ont les significations données précédemment avec le chloroacétonitrile de manière à obtenir un composé de formule (III) :

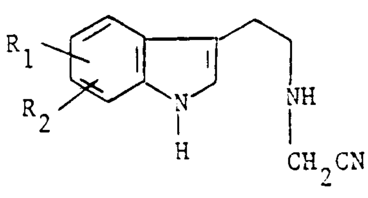

(III)

que l'on réduit en un composé de formule (IV) :

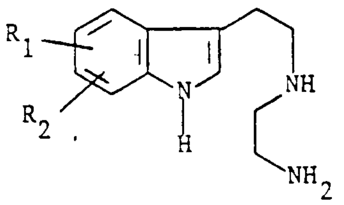

(IV)

que l'on condense avec un maléate ou un fumarate d'alcoyle de formule

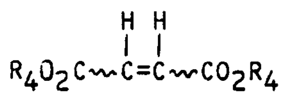

dans laquelle $R_4$ est un radical alcoyle renfermant de 1 à 4 atomes de carbone pour obtenir un composé de formule (V) :

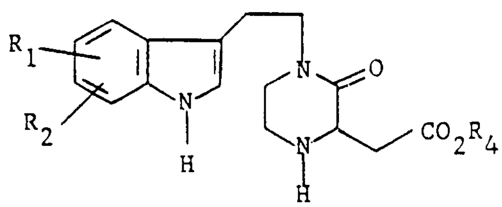

(V)

sur lequel on fait réagir un réactif susceptible d'introduire un radical R', R' ayant les valeurs indiquées pour R à l'exception de la valeur hydrogène, pour obtenir un composé de formule (VI) :

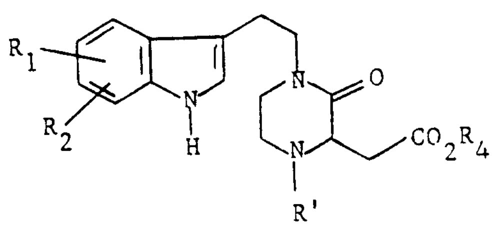

(VI)

sur lequel on fait réagir un agent de cyclisation pour obtenir un composé de formule (VII) :

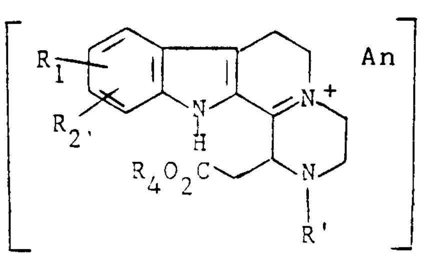

(VII)

dans lequel $R_1$, $R_2$, $R_4$ et R' ont les significations indiquées précédemment et An représente un anion dérivé d'un acide fort et que, soit l'on réduit le composé de formule (VII) en composé de formule (VIII) dans laquelle les deux atomes d'hydrogène en position 3 et 16 sont en position cis :

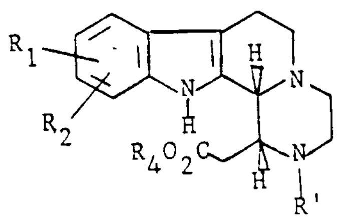

(VIII)

dont on élimine le cas échéant le groupement R' dans le cas où celui-ci représente un radical benzoyle ou alcoylcarbonyle et que l'on cyclise pour obtenir un composé de formule

$$(I_{A_{1b}})$$

correspondant à un produit de formule (I) dans laquelle :

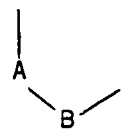

représente

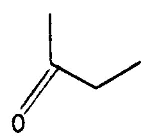

les deux atomes d'hydrogène en position 3 et 16 sont en position cis et R représente un atome d'hydrogène et composé de formule

$$(I_{A_{1b}})$$

que l'on soumet le cas échéant à l'action d'un réactif susceptible d'introduire un radical R', R' ayant toutes les valeurs indiquées pour R à l'exception de la valeur hydrogène, pour obtenir un composé de formule

$$(I_{A_{1c}})$$

dans laquelle R a la signification indiquée ci-dessus pour R', soit l'on cyclise le composé de formule (VII) en composé de formule (IX) :

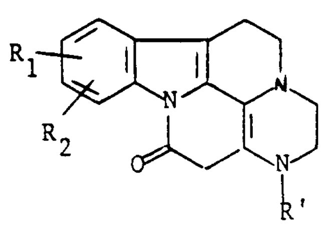

(IX)

que l'on réduit pour obtenir un composé de formule

$(I_{A_{2a}})$

correspondant à un produit de formule (I) dans laquelle :

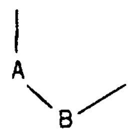

représente

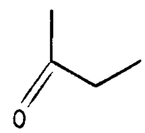

les deux atomes d'hydrogène en position 3 et 16 sont en position trans et R a la signification indiquée ci-dessus pour R', dont on élimine le cas échéant, le groupement R' dans le cas où celui-ci représente un radical benzoyle ou alcoylcarbonyle, pour obtenir un composé de formule

$(I_{A_{2b}})$

dans laquelle R est un atome d'hydrogène, que l'on soumet, le cas échéant, à l'action d'un réactif susceptible d'introduire un radical R', R' ayant les valeurs indiquées pour R à l'exception de la valeur hydrogène pour obtenir un composé de formule

$(I_{A_{2c}})$

dans laquelle R a la signification indiquée ci-dessus pour R', lesdits composés de formules

$(I_{A_{1b}})$,

$(I_{A_{1c}})$, $(I_{A_{2a}})$, $(I_{A_{2b}})$ et $(I_{A_{2c}})$

étant, si désiré, réduits en composés correspondants de formules

$(I_{B_{1b}})$, $(I_{B_{1c}})$, $(I_{B_{2a}})$,

$(I_{B_{2b}})$ et $(I_{B_{2c}})$

représentant les composés de formule (I) dans laquelle :

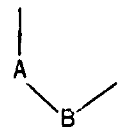

représente

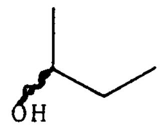

lesdits composés de formules

$(I_{B_{1b}})$, $(I_{B_{1c}})$, $(I_{B_{2a}})$,

$(I_{B_{2b}})$, $(I_{B_{2c}})$

étant, si désiré, soumis à l'action d'un agent déshydratant pour obtenir les composés correspondants de formules

$(I_{C_{1b}})$, $(I_{C_{1c}})$, $(I_{C_{2a}})$, $(I_{C_{2b}})$ et $(I_{C_{2c}})$

représentant les composés de formule (I) dans laquelle :

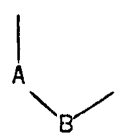

représente

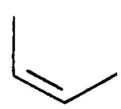

lesdits composés de formules

$(I_{C_{1b}})$, $(I_{C_{1c}})$, $(I_{C_{2a}})$,

$(I_{C_{2b}})$ et $(I_{C_{2c}})$

étant, si désire, soumis à l'action d'un agent de réduction, pour obtenir les composés correspondants de formules

$(I_{D_{1b}})$,

$(I_{D_{1c}})$, $(I_{D_{2a}})$, $(I_{D_{2b}})$ et $(I_{D_{2c}})$

eprésentant les composés de formule (I) dans laquelle :

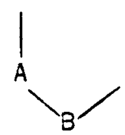

représente

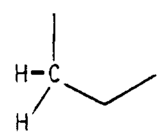

et traite si désiré tous les produits de formule (I) obtenus par un acide minéral ou organique pour en former les sels.

**2.** Procédé selon la revendication 1, caractérisé en ce que l'on utilise au départ un composé de formule (II) dans laquelle $R_1$ et $R_2$ représentent un atome d'hydrogène, un radical méthyle, éthyle, méthoxy ou éthoxy, unatome de chlore, un radical hydroxy, trifluorométhyle ou nitro et un réactif susceptible d'introduire un radical R′ tel que R′ représente un radical méthyle, éthyle ou propyle, benzyle, phénéthyle ou benzoyle.

**3.** Procédé selon la revendication 1 ou 2, caractérisé en ce que l'on prépare des composés de formule (I) dans laquelle $R_2$ représente un atome d'hydrogène, $R_1$ représente un atome d'hydrogène, un radical méthoxy ou éthoxy, un atome de chlore sous toutes les formes isomères possibles ou racémiques ou optiquement actives, ainsi que les sels d'addition avec les acides minéraux ou organiques.

**4.** Procédé selon l'une quelconque des revendications 1 à 3, caractérisé en ce que l'on prépare des composés de formule (I), dans laquelle l'atome d'hydrogène en position 3 et l'atome d'hydrogène en position 16 sonttrans sous toutes les formes isomères possibles racémiques ou optiquement actives ainsi que les sels d'addition avec les acides minéraux ou organiques.

**5.** Procédé selon la revendication 1, caractérisé en ce que l'on prépare :
- La (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine, et leurs sels d'addition avec les acides.

**Revendications pour l'Etats contractant suivant : GR**

**1.** Procédé pour préparer les composés de formule (I) :

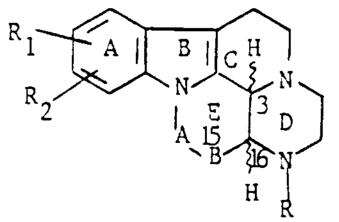

(I)

dans laquelle $R_1$ et $R_2$, identiques ou différents, représentent un atome d'hydrogène, un atome d'halogène, un radical alcoyle renfermant de 1 à 5 atomes de carbone, alcoxy renfermant de 1 à 5 atomes de carbone, un radical hydroxy, trifluorométhyle ou nitro, R représente un atome d'hydrogène, un radical alcoyle renfermant de 1 à 5 atomes de carbone, un radical arylalcoyle renfermant de 7 à 12 atomes de carbone, un radical benzoyle ou alcoylcarbonyle dans lequel le radical alcoyle renferme de 1 à 12 atomes de carbone, et dans laquelle le groupement :

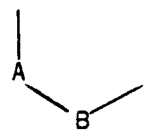

représente soit

i) 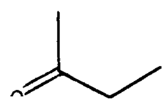

soit

ii) 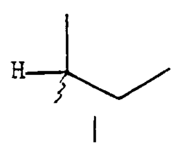 ,

soit

iii) 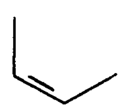 ,

soit

iiii) 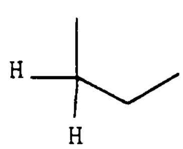

lesdits produits de formule (I) étant sous toutes les formes isomères possibles racémiques ou

optiquement actives, ainsi que les sels d'addition avec les acides minéraux ou organiques desdits produits de formule (I), caractérisé en ce que l'on fait réagir un composé de formule (II) :

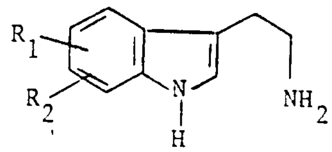

(II)

dans laquelle $R_1$ et $R_2$ ont les significations données précédemment avec le chloroacétonitrile de manière à obtenir un composé de formule (III) :

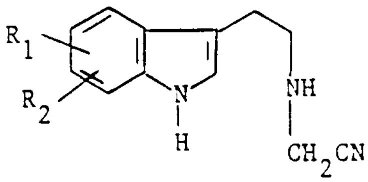

(III)

que l'on réduit en un composé de formule (IV) :

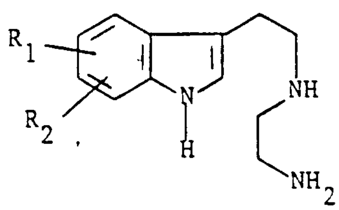

(IV)

que l'on condense avec un maléate ou un fumarate d'alcoyle de formule

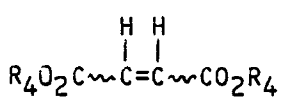

dans laquelle $R_4$ est un radical alcoyle renfermant de 1 à 4 atomes de carbone pour obtenir un composé de formule (V) :

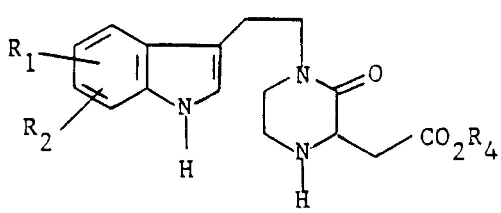

(V)

sur lequel on fait réagir un réactif susceptible d'introduire un radical R', R' ayant les valeurs indiquées pour R à l'exception de la valeur hydrogène, pour obtenir un composé de formule (VI) :

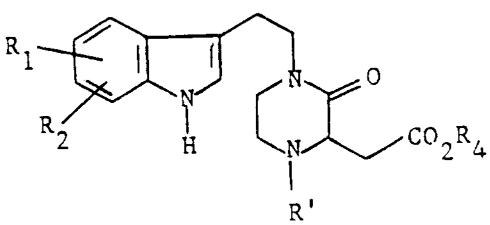

(VI)

sur lequel on fait réagir un agent de cyclisation pour obtenir un composé de formule (VII) :

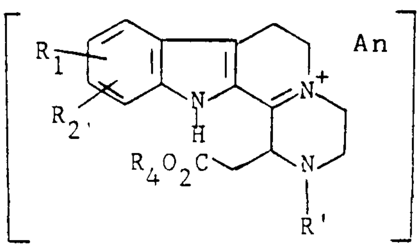

(VII)

dans lequel $R_1$, $R_2$, $R_4$ et R' ont les significations indiquées précédemment et An représente un anion dérivé d'un acide fort et que, soit l'on réduit le composé de formule (VII) en composé de formule (VIII) dans laquelle les deux atomes d'hydrogène en position 3 et 16 sont en position cis :

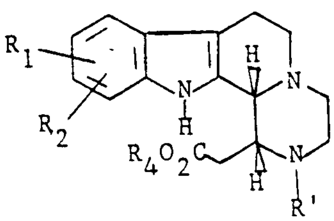

(VIII)

dont on élimine le cas échéant le groupement R' dans le cas où celui-ci représente un radical benzoyle ou alcoylcarbonyle et que l'on cyclise pour obtenir un composé de formule

$(I_{A_{1b}})$

correspondant à un produit de formule (I) dans laquelle :

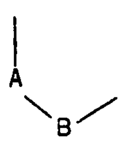

représente

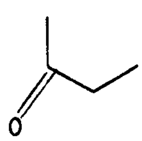

les deux atomes d'hydrogène en position 3 et 16 sont en position cis et R représente un atome

d'hydrogène et composé de formule

$(I_{A_{1b}})$

que l'on soumet le cas échéant à l'action d'un réactif susceptible d'introduire un radical, R', R' ayant toutes les valeurs indiquées pour R à l'exception de la valeur hydrogène, pour obtenir un composé de formule

$(I_{A_{1c}})$

dans laquelle R a la signification indiquée ci-dessus pour R', <u>soit</u> l'on cyclise le composé de formule (VII) en composé de formule (IX) :

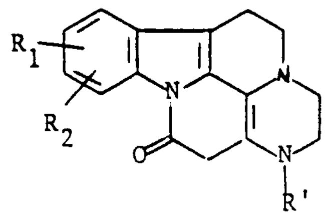

(IX)

que l'on réduit pour obtenir un composé de formule

$(I_{A_{2a}})$

correspondant à un produit de formule (I) dans laquelle :

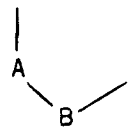

représente

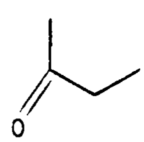

les deux atomes d'hydrogène en position 3 et 16 sont en position trans et R a la signification indiquée ci-dessus pour R', dont on élimine le cas échéant, le groupement R' dans le cas où celui-ci représente un radical benzoyle ou alcoylcarbonyle, pour obtenir un composé de formule

$(I_{A_{2b}})$

dans laquelle R est un atome d'hydrogène, que l'on soumet, le cas échéant, à l'action d'un réactif susceptible d'introduire un radical R', R' ayant les valeurs indiquées pour R à l'exception de la valeur hydrogène pour obtenir un composé de formule

$(I_{A_{2c}})$

dans laquelle R a la signification indiquée ci-dessus pour R', lesdits composés de formules

$(I_{A_{1b}})$ ,

$(I_{A_{1c}})$, $(I_{A_{2a}})$, $(I_{A_{2b}})$ et $(I_{A_{2c}})$

étant, si désiré, réduits en composés correspondants de formules

$(I_{B_{1b}})$, $(I_{B_{1c}})$, $(I_{B_{2a}})$,

$(I_{B_{2b}})$ et $(I_{B_{2c}})$

représentant les coposés de formule (I) dans laquelle :

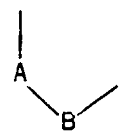

représente

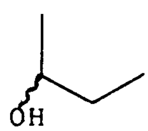

lesdits composés de formules

$(I_{B_{1b}})$, $(I_{B_{1c}})$, $(I_{B_{2a}})$,

$(I_{B_{2b}})$, $(I_{B_{2c}})$

étant, si désiré, soumis à l'action d'un agent déshydratant pour obtenir les composés correspondants de formules

$(I_{C_{1b}})$, $(I_{C_{1c}})$, $(I_{C_{2a}})$, $(I_{C_{2b}})$ et $(I_{C_{2c}})$

représentant les composés de formule (I) dans laquelle :

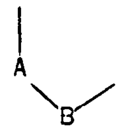

représente

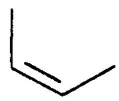

lesdits composés de formules

$(I_{C_{1b}})$, $(I_{C_{1c}})$, $(I_{C_{2a}})$,

$(I_{C_{2b}})$ et $(I_{C_{2c}})$

étant, si désire, soumis à l'action d'un agent de réduction, pour obtenir les composés correspondants de formules

$(I_{D_{1b}})$,

$(I_{D_{1c}})$, $(I_{D_{2a}})$, $(I_{D_{2b}})$ et $(I_{D_{2c}})$

eprésentant les composés de formule (I) dans laquelle :

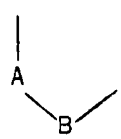

représente

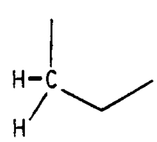

et traite si désiré tous les produits de formule (I) obtenus par un acide minéral ou organique pour en former les sels.

**2.** Procédé selon la revendication 1, caractérisé en ce que l'on utilise au départ un composé de formule (II) dans laquelle $R_1$ et $R_2$ représentent un atome d'hydrogène, un radical méthyle, éthyle, méthoxy ou éthoxy, unatome de chlore, un radical hydroxy, trifluorométhyle ou nitro et un réactif susceptible

d'introduire un radical R′ tel que R′ représente un radical méthyle, éthyle ou propyle, benzyle, phénéthyle ou benzoyle.

**3.** Procédé selon la revendication 1 ou 2, caractérisé en ce que l'on prépare des composés de formule (I) dans laquelle $R_2$ représente un atome d'hydrogène, $R_1$ représente un atome d'hydrogène, un radical méthoxy ou éthoxy, un atome de chlore sous toutes les formes isomères possibles ou racémiques ou optiquement actives, ainsi que les sels d'addition avec les acides minéraux ou organiques.

**4.** Procédé selon l'une quelconque des revendications 1 à 3, caractérisé en ce que l'on prépare des composés de formule (I), dans laquelle l'atome d'hydrogène en position 3 et l'atome d'hydrogène en position 16 sonttrans sous toutes les formes isomères possibles racémiques ou optiquement actives ainsi que les sels d'addition avec les acides minéraux ou organiques.

**5.** Procédé selon la revendication 1, caractérisé en ce que l'on prépare :
- La (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine, et leurs sels d'addition avec les acides.

**6.** Procédé de préparation de compositions pharmaceutiques caractérisé en ce que l'on met, à titre de principe actif, l'un au moins des composés de formule (I), tels que définis à la revendication 1, ou l'un au moins de leurs sels d'addition avec les acides minéraux ou organiques pharmaceutiquement acceptables, sous une forme destinée à cet usage.

**7.** Procédé selon la revendication 6, caractérisé en ce que le composé de formule (I) est choisi parmi :
- La (16alpha) (±) 17-méthyl 17-aza 20,21-dinoréburnaménin 14 (15H)-one,
- la (16alpha) (±) 17-propyl 17-aza 20,21-dinoréburnaménin 14 (15H)-one,
- la (16alpha) (±) 17-aza 20,21-dinoréburnaménine,
- la (16alpha) (±) 14,15-dihydro 17-méthyl 17-aza 20,21-dinoréburnaménine, et leurs sels d'addition avec les acides.

**8.** A titre de produits industriels, les composés de formules (III), (IV') correspondant à la formule (IV) dans laquelle lorsque l'un de $R_1$ ou $R_2$ représente un atome d'hydrogène, l'autre de $R_1$ ou $R_2$ ne représente pas un radical méthoxy, les composés (V), (VI), (VII), (VIII) et (IX).

**Claims**

**Claims for the following Contracting States : AT, BE, DE, FR, GB, IT, LI, LU, NL, SE**

**1.** Compounds of formula (I):

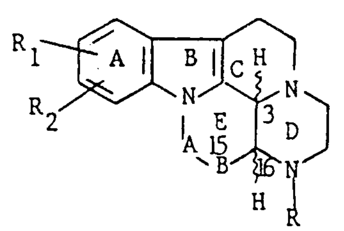

(I)

in which $R_1$ and $R_2$, identical or different, represent a hydrogen atom, a halogen atom, an alkyl radical containing 1 to 5 carbon atoms, an alkoxy radical containing 1 to 5 carbon atoms, a hydroxy, trifluoromethyl or nitro radical, R represents a hydrogen atom, an alkyl radical containing 1 to 5 carbon atoms, an arylalkyl radical containing 7 to 12 carbon atoms, a benzoyl or alkylcarbonyl radical in which the alkyl radical contains 1 to 12 carbon atoms, and in which the

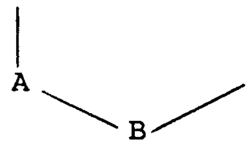

group: represents either

i) 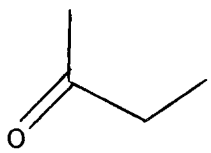

or

ii) 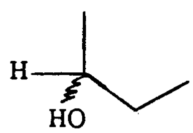 ,

or

iii) 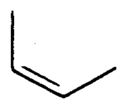 ,

or

iiii) 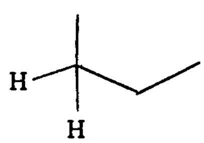

the said products of formula (I) being in all the possible racemic or optically active isomer forms, as well as the addition salts with mineral or organic acids of said products of formula (I).

**2.** Compounds of formula (I), as defined in claim 1, characterized in that $R_1$ and $R_2$ represent a hydrogen atom, a methyl, ethyl, methoxy or ethoxy radical, a chlorine atom, a hydroxy, trifluoromethyl or nitro radical, and R represents a hydrogen atom, a methyl, ethyl or propyl, benzyl, phenethyl or benzoyl radical, in all the possible racemic or optically active isomer forms, as well as the addition salts with mineral or organic acids.

**3.** Compounds of formula (I), as defined in claims 1 and 2, characterized in that $R_2$ represents a hydrogen atom, $R_1$ represents a hydrogen atom, a methoxy or ethoxy radical, a chlorine atom, in all the possible racemic or optically active isomer forms, as well as the addition salts with mineral or organic acids.

**4.** Compounds of formula (I), as defined in claims 1 to 3, characterized in that the hydrogen atom in position 3 and the hydrogen atom in position 16 are trans in all the possible racemic or optically active isomer forms, as well as the addition salts with mineral or organic acids.

**5.**

- (16alpha) (±) 17-methyl-17-aza-20,21-dinoreburnamenin-14-(15H)-one,
- (16alpha) (±) 17-propyl-17-aza-20,21-dinoreburnamenin-14-(15H)-one,

- (16alpha) (±) 17-aza-20,21-dinoreburnamenine,
- (16alpha) (±) 14,15-dihydro-17-methyl-17-aza-20,21-dinoreburnamenine, and their addition salts with acids.

**6.** Preparation process for the compounds of formula (I), as defined in claim 1, characterized in that a compound of formula (II):

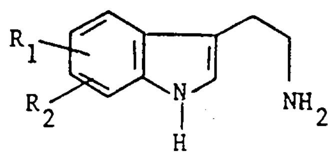

(II)

in which $R_1$ and $R_2$ have the meanings given above, is reacted with chloroacetonitrile so as to obtain a compound of formula (III):

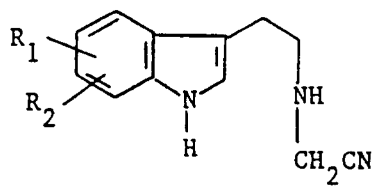

(III)

which is reduced into a compound of formula (IV):

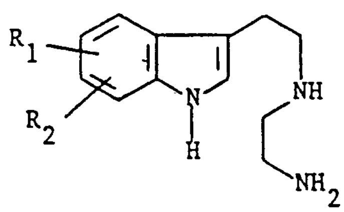

(IV)

which is condensed with an alkyl maleate or fumarate of formula

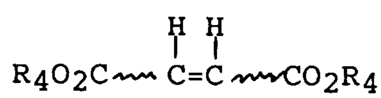

in which $R_4$ is an alkyl radical containing 1 to 4 carbon atoms in order to obtain a compound of formula (V):

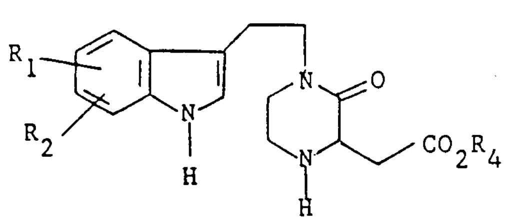

(V)

on which a reagent is reacted which is capable of introducing an R' radical, R' having the values indicated for R with the exception of the hydrogen value, in order to obtain a compound of formula (VI):

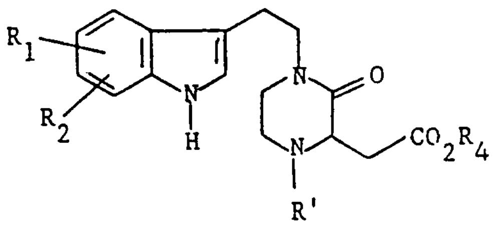

(VI)

on which a cyclization agent is reacted in order to obtain a compound of formula (VII):

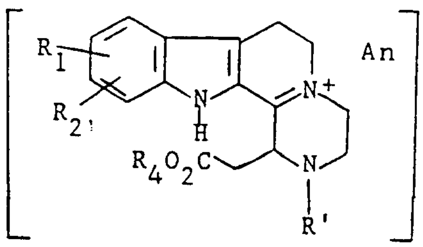

(VII)

in which $R_1$, $R_2$, $R_4$ and R' have the meanings indicated previously and An represents an anion derivative of a strong acid and in that, either the compound of formula (VII) is reduced into a compound of formula (VIII) in which the two hydrogen atoms in position 3 and 16 are in cis position:

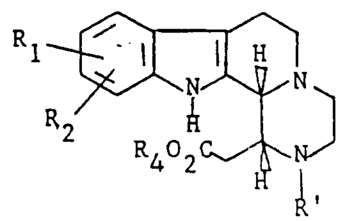

(VIII)

the R' group of which is eliminated if appropriate in the case where the latter represents a benzoyl or alkylcarbonyl radical and which is cyclized in order to obtain a compound of formula ($I_{A1b}$) corresponding to a product of formula (I) in which:

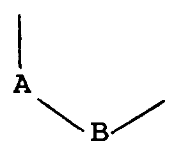

represents

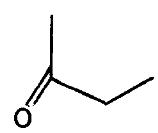

the two hydrogen atoms in position 3 and 16 are in cis position and R represents a hydrogen atom and which compound of formula ($I_{A1b}$) is subjected, if appropriate, to the action of a reagent capable of introducing an R' radical, R' having all the values indicated for R with the exception of the hydrogen

value, in order to obtain a compound of formula ($I_{A1c}$) in which R has the meaning indicated above for R', or the compound of formula (VII) is cyclized into a compound of formula (IX):

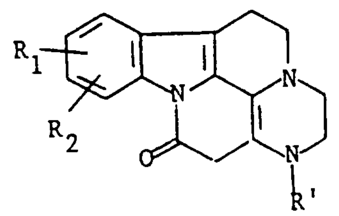

(IX)

which is reduced in order to obtain a compound of formula ($I_{A2a}$) corresponding to a product of formula (I) in which:

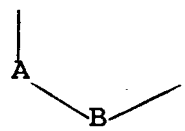

represents

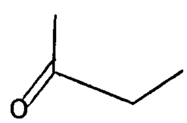

the two hydrogen atoms in position 3 and 16 are in trans position and R has the meaning indicated above for R', the R' group of which is eliminated if appropriate in the case where the latter represents a benzoyl or alkylcarbonyl radical, in order to obtain a compound of formula ($I_{A2b}$) in which R is a hydrogen atom, which is subjected, if appropriate, to the action of a reagent capable of introducing an R' radical, R' having the values indicated for R with the exception of the hydrogen value, in order to obtain a compound of formula ($I_{A2c}$) in which R has the meaning indicated above for R', the said compounds of formulae ($I_{A1b}$), ($I_{A1c}$), ($I_{A2a}$), ($I_{A2b}$) and ($I_{A2c}$) being, if desired, reduced into corresponding compounds of formulae ($I_{B1b}$), ($I_{B1c}$), ($I_{B2a}$), ($I_{B2b}$) and ($I_{B2c}$) representing the compounds of formula (I) in which:

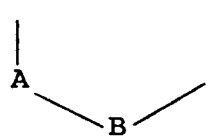

represents

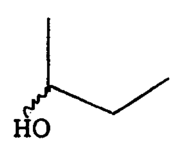

the said compounds of formulae ($I_{B1b}$), ($I_{B1c}$), ($I_{B2a}$), ($I_{B2b}$), ($I_{B2c}$) being, if desired, subjected to the action of a dehydrating agent in order to obtain the corresponding compounds of formulae ($I_{C1b}$), ($I_{C1c}$), ($I_{C2a}$), ($I_{C2b}$) and ($I_{C2c}$) representing the compounds of formula (I) in which:

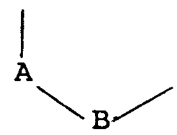

represents

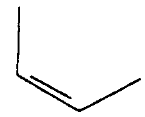

the said compounds of formulae ($I_{C1b}$), ($I_{C1c}$), ($I_{C2a}$), ($I_{C2b}$) and ($I_{C2c}$) being, if desired, subjected to the action of a reducing agent, in order to obtain the corresponding compounds of formulae ($I_{D1b}$), ($I_{D1c}$), ($I_{D2a}$), ($I_{D2b}$) and ($I_{D2c}$) representing the compounds of formula (I) in which:

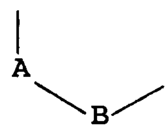

represents

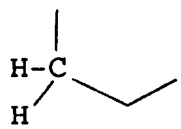

and if desired all the products of formula (I) obtained are treated with a mineral or organic acid in order to form the salts.

**7.** As medicaments, the compounds as defined by formula (I) of claim 1, the said products of formula (I) being in all the possible racemic or optically active isomer forms, as well as the addition salts with pharmaceutically acceptable mineral or organic acids.

**8.** As medicaments, the compounds as defined in claims 2 to 4.

**9.** As medicaments, the compounds of claim 5.

**10.** The pharmaceutical compositions containing as active ingredient at least one of the medicaments as defined in any one of claims 7 to 9.

**11.** As industrial products, the compounds of formulae (III), (IV') corresponding to formula (IV) in which when one of $R_1$ or $R_2$ represents a hydrogen atom, the other of $R_1$ or $R_2$ does not represent a methoxy radical, the compounds (V), (VI), (VII), (VIII) and (IX).

**Claims for the following Contracting State: ES**

**1.** Process for preparing the compounds of formula (I):

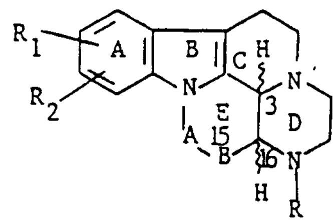

(I)

in which $R_1$ and $R_2$, identical or different, represent a hydrogen atom, a halogen atom, an alkyl radical containing 1 to 5 carbon atoms, an alkoxy radical containing 1 to 5 carbon atoms, a hydroxy, trifluoromethyl or nitro radical, R represents a hydrogen atom, an alkyl radical containing 1 to 5 carbon atoms, an arylalkyl radical containing 7 to 12 carbon atoms, a benzoyl or alkylcarbonyl radical in which the alkyl radical contains 1 to 12 carbon atoms, and in which the group:

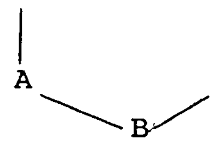

represents either

i) 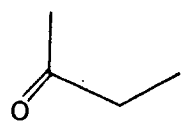

or

ii) 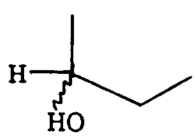 ,

or

iii) 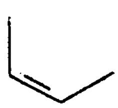 ,

or

iiii) 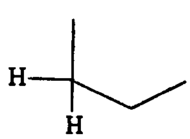

the said products of formula (I) being in all the possible racemic or optically active isomer forms, as well as the addition salts with mineral or organic acids of said products of formula (I), characterized in that a compound of formula (II):

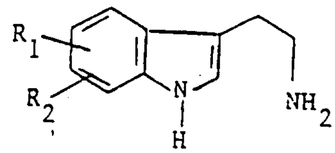

(II)

in which $R_1$ and $R_2$ have the meanings given previously, are reacted with chloroacetonitrile so as to obtain a compound of formula (III):

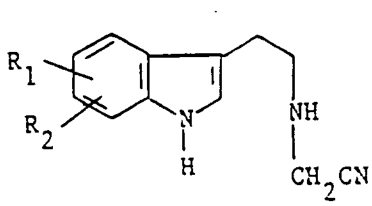

(III)

which is reduced into a compound of formula (IV):

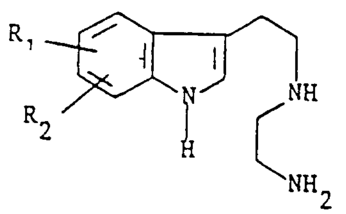

(IV)

which is condensed with an alkyl maleate or fumarate of formula

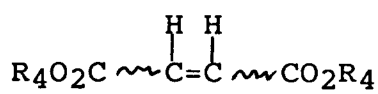

in which $R_4$ is an alkyl radical containing 1 to 4 carbon atoms in order to obtain a compound of formula (V):

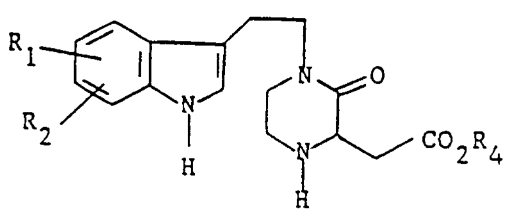

(V)

on which a reagent is reacted which is capable of introducing an R' radical, R' having the values indicated for R with the exception of the hydrogen value, in order to obtain a compound of formula (VI):

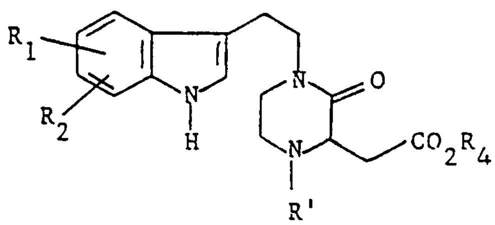

(VI)

on which a cyclization agent is reacted in order to obtain a compound of formula (VII):

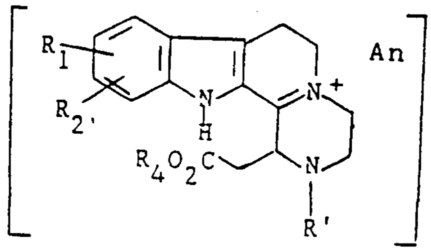

(VII)

in which $R_1$, $R_2$, $R_4$ and R' have the meanings indicated previously and An represents an anion derivative of a strong acid and in that, either the compound of formula (VII) is reduced into a compound of formula (VIII) in which the two hydrogen atoms in position 3 and 16 are in cis position:

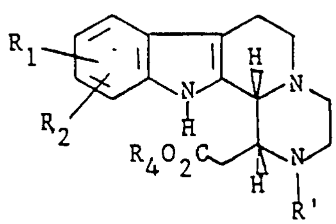

(VIII)

the R' group of which is eliminated if appropriate in the case where the latter represents a benzoyl or alkylcarbonyl radical and which is cyclized in order to obtain a compound of formula ($I_{A1b}$) corresponding to a product of formula (I) in which:

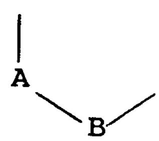

represents

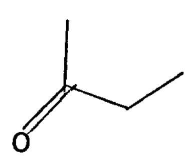

the two hydrogen atoms in position 3 and 16 are in cis position and R represents a hydrogen atom and which compound of formula ($I_{A1b}$) is subjected, if appropriate, to the action of a reagent capable of introducing an R' radical, R' having all the values indicated for R with the exception of the hydrogen value, in order to obtain a compound of formula ($I_{A1c}$) in which R has the meaning indicated above for

R', or the compound of formula (VII) is cyclized into a compound of formula (IX):

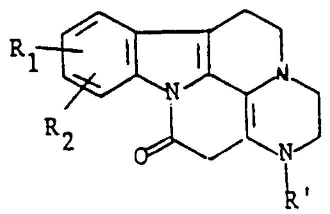

(IX)

which is reduced in order to obtain a compound of formula ($I_{A2a}$) corresponding to a product of formula (I) in which:

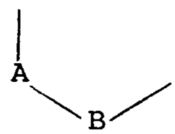

represents

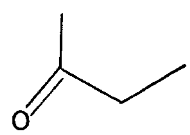

the two hydrogen atoms in position 3 and 16 are in trans position and R has the meaning indicated above for R', the R' group of which is eliminated if appropriate in the case where the latter represents a benzoyl or alkylcarbonyl radical, in order to obtain a compound of formula ($I_{A2b}$) in which R is a hydrogen atom, which is subjected, if appropriate, to the action of a reagent capable of introducing an R' radical, R' having the values indicated for R with the exception of the hydrogen value, in order to obtain a compound of formula ($I_{A2c}$) in which R has the meaning indicated above for R', the said compounds of formulae ($I_{A1b}$), ($I_{A1c}$), ($I_{A2a}$), ($I_{A2b}$) and ($I_{A2c}$) being, if desired, reduced into corresponding compounds of formulae ($I_{B1b}$), ($I_{B1c}$), ($I_{B2a}$), ($I_{B2b}$) and ($I_{B2c}$) representing the compounds of formula (I) in which:

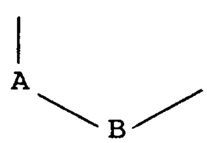

represents

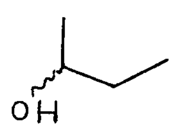

the said compounds of formulae ($I_{B1b}$), ($I_{B1c}$), ($I_{B2a}$), ($I_{B2b}$), ($I_{B2c}$) being, if desired, subjected to the action of a dehydrating agent in order to obtain the corresponding compounds of formulae ($I_{C1b}$), ($I_{C1c}$), ($I_{C2a}$), ($I_{C2b}$) and ($I_{C2c}$) representing the compounds of formula (I) in which:

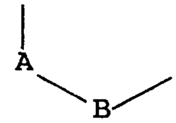

represents

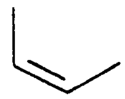

the said compounds of formulae ($I_{C1b}$), ($I_{C1c}$), ($I_{C2a}$), ($I_{C2b}$) and ($I_{C2c}$) being, if desired, subjected to the action of a reducing agent, in order to obtain the corresponding compounds of formulae ($I_{D1b}$), ($I_{D1c}$), ($I_{D2a}$), ($I_{D2b}$) and ($I_{D2c}$) representing the compounds of formula (I) in which:

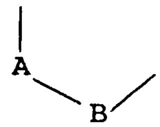

represents

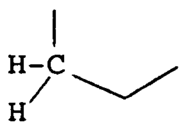

and if desired all the products of formula (I) obtained are treated with a mineral or organic acid in order to form the salts.

**2.** Process according to claim 1, characterized in that at the start a compound of formula (II) is used in which $R_1$ and $R_2$ represent a hydrogen atom, a methyl, ethyl, methoxy or ethoxy radical, a chlorine atom, a hydroxy, trifluoromethyl or nitro radical and a reagent capable of introducing an R' radical such that R' represents a methyl, ethyl or propyl, benzyl, phenethyl or benzoyl radical.

**3.** Process according to claim 1 or 2, characterized in that compounds of formula (I) are prepared in which $R_2$ represents a hydrogen atom, $R_1$ represents a hydrogen atom, a methoxy or ethoxy radical, a chlorine atom, in all the possible racemic or optically active isomer forms, as well as the addition salts with mineral or organic acids.

**4.** Process according to any one of claims 1 to 3, characterized in that compounds of formula (I) are prepared in which the hydrogen atom in position 3 and the hydrogen atom in position 16 are trans in all the possible racemic or optically active isomer forms as well as the addition salts with mineral or organic acids.

**5.** Process according to claim 1, characterized in that the following are prepared:
- (16alpha) (±) 17-methyl-17-aza-20,21-dinoreburnamenin-14-(15H)-one,
- (16alpha) (±) 17-propyl-17-aza-20,21-dinoreburnamenin-14-(15H)-one,
- (16alpha) (±) 17-aza-20,21-dinoreburnamenine,
- (16alpha) (±) 14,15-dihydro-17-methyl-17-aza-20,21-dinoreburnamenine, and their addition salts with acids.

**Claims for the following Contracting State: GR**

**1.** Process for preparing the compounds of formula (I):

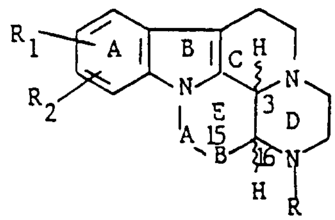

(I)

in which $R_1$ and $R_2$, identical or different, represent a hydrogen atom, a halogen atom, an alkyl radical containing 1 to 5 carbon atoms, an alkoxy radical containing 1 to 5 carbon atoms, a hydroxy, trifluoromethyl or nitro radical, R represents a hydrogen atom, an alkyl radical containing 1 to 5 carbon atoms, an arylalkyl radical containing 7 to 12 carbon atoms, a benzoyl or alkylcarbonyl radical in which the alkyl radical contains 1 to 12 carbon atoms, and in which the group:

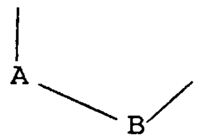

represents either

i) 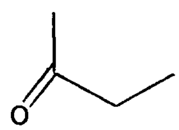

or

ii) 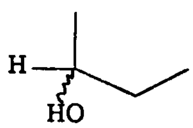 ,

or

iii) 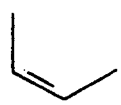 ,

or

iiii) 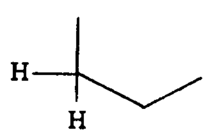

the said products of formula (I) being in all the possible racemic or optically active isomer forms, as well as the addition salts with mineral or organic acids of said products of formula (I), characterized in that a compound of formula (II):

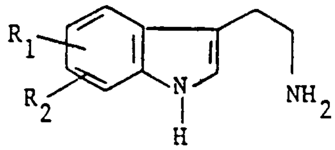

(II)

in which $R_1$ and $R_2$ have the meanings given above, are reacted with chloroacetonitrile so as to obtain a compound of formula (III):

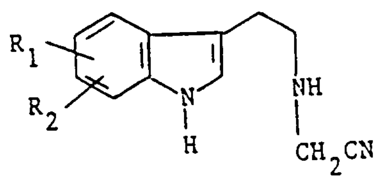

(III)

which is reduced into a compound of formula (IV):

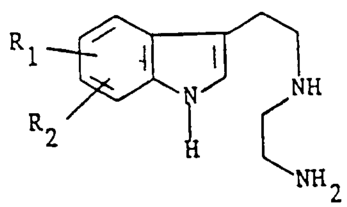

(IV)

which is condensed with an alkyl maleate or fumarate of formula

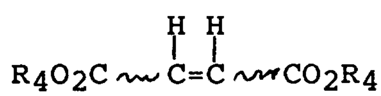

in which $R_4$ is an alkyl radical containing 1 to 4 carbon atoms in order to obtain a compound of formula (V):

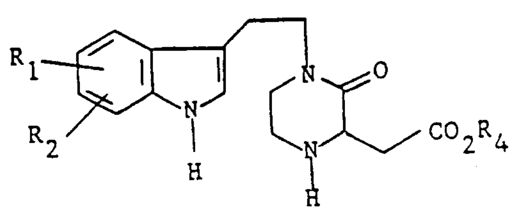

(V)

on which a reagent is reacted which is capable of introducing an R' radical, R' having the values indicated for R with the exception of the hydrogen value, in order to obtain a compound of formula (VI):

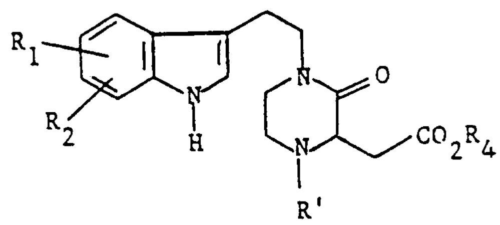

(VI)

on which a cyclization agent is reacted in order to obtain a compound of formula (VII):

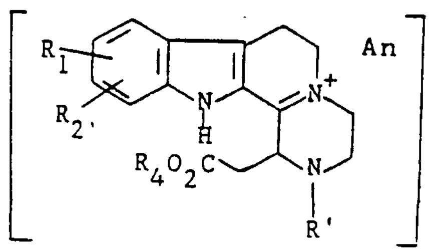

(VII)

in which $R_1$, $R_2$, $R_4$ and R' have the meanings indicated previously and An represents an anion derivative of a strong acid and in that, either the compound of formula (VII) is reduced into a compound of formula (VIII) in which the two hydrogen atoms in position 3 and 16 are in cis position:

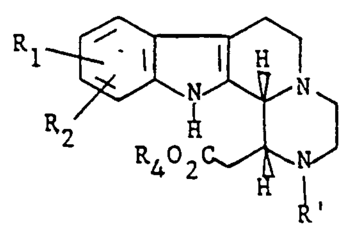

(VIII)

the R' group of which is eliminated if appropriate in the case where the latter represents a benzoyl or alkylcarbonyl radical and which is cyclized in order to obtain a compound of formula ($I_{A1b}$) corresponding to a product of formula (I) in which:

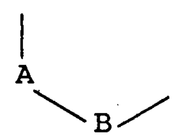

represents

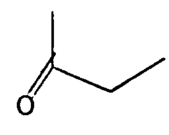

the two hydrogen atoms in position 3 and 16 are in cis position and R represents a hydrogen atom and which compound of formula ($I_{A1b}$) is subjected, if appropriate, to the action of a reagent capable of introducing an R' radical, R' having all the values indicated for R with the exception of the hydrogen value, in order to obtain a compound of formula ($I_{A1c}$) in which R has the meaning indicated above for R', or the compound of formula (VII) is cyclized into a compound of formula (IX):

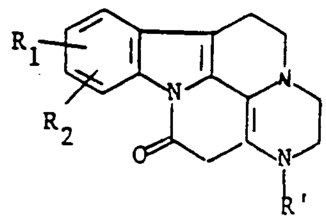

(IX)

which is reduced in order to obtain a compound of formula ($I_{A2a}$) corresponding to a product of formula (I) in which:

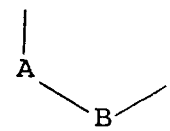

represents

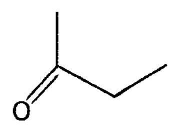

the two hydrogen atoms in position 3 and 16 are in trans position and R has the meaning indicated above for R', the R' group of which is eliminated if appropriate in the case where the latter represents a benzoyl or alkylcarbonyl radical, in order to obtain a compound of formula ($I_{A2b}$) in which R is a hydrogen atom, which is subjected, if appropriate, to the action of a reagent capable of introducing an R' radical, R' having the values indicated for R with the exception of the hydrogen value, in order to obtain a compound of formula ($I_{A2c}$) in which R has the meaning indicated above for R', the said compounds of formulae ($I_{A1b}$), ($I_{A1c}$), ($I_{A2a}$), ($I_{A2b}$) and ($I_{A2c}$) being, if desired, reduced into corresponding compounds of formulae ($I_{B1b}$), ($I_{B1c}$), ($I_{B2a}$), ($I_{B2b}$) and ($I_{B2c}$) representing the compounds of formula (I) in which:

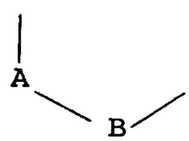

represents

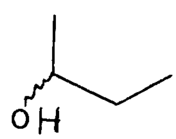

the said compounds of formulae ($I_{B1b}$), ($I_{B1c}$), ($I_{B2a}$), ($I_{B2b}$), ($I_{B2c}$) being, if desired, subjected to the action of a dehydrating agent in order to obtain the corresponding compounds of formulae ($I_{C1b}$), ($I_{C1c}$), ($I_{C2a}$), ($I_{C2b}$) and ($I_{C2c}$) representing the compounds of formula (I) in which:

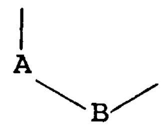

represents

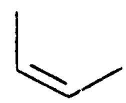

the said compounds of formulae ($I_{C1b}$), ($I_{C1c}$), ($I_{C2a}$), ($I_{C2b}$) and ($I_{C2c}$) being, if desired, subjected to the action of a reducing agent, in order to obtain the corresponding compounds of formulae ($I_{D1b}$), ($I_{D1c}$), ($I_{D2a}$), ($I_{D2b}$) and ($I_{D2c}$) representing the compounds of formula (I) in which:

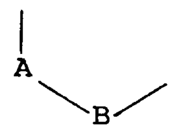

represents

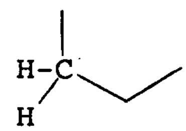

and if desired all the products of formula (I) obtained are treated with a mineral or organic acid in order to form the salts.

**2.** Process according to claim 1, characterized in that at the start a compound of formula (II) is used in which $R_1$ and $R_2$ represent a hydrogen atom, a methyl, ethyl, methoxy or ethoxy radical, a chlorine atom, a hydroxy, trifluoromethyl or nitro radical and a reagent capable of introducing an R' radical such that R' represents a methyl, ethyl or propyl, benzyl, phenethyl or benzoyl radical.

**3.** Process according to claim 1 or 2, characterized in that compounds of formula (I) are prepared in which $R_2$ represents a hydrogen atom, $R_1$ represents a hydrogen atom, a methoxy or ethoxy radical, a chlorine atom, in all the possible racemic or optically active isomer forms, as well as the addition salts with mineral or organic acids.

**4.** Process according to any one of claims 1 to 3, characterized in that compounds of formula (I) are prepared in which the hydrogen atom in position 3 and the hydrogen atom in position 16 are trans in all the possible racemic or optically active isomer forms as well as the addition salts with mineral or organic acids.

**5.** Process according to claim 1, characterized in that the following are prepared:
- (16alpha) (±) 17-methyl-17-aza-20,21-dinoreburnamenin-14-(15H)-one,
- (16alpha) (±) 17-propyl-17-aza-20,21-dinoreburnamenin-14-(15H)-one,
- (16alpha) (±) 17-aza-20,21-dinoreburnamenine,
- (16alpha) (±) 14,15-dihydro-17-methyl-17-aza-20,21-dinoreburnamenine, and their addition salts with acids.

6. Preparation process for pharmaceutical compositions characterized in that at least one of the compounds of formula (I), as defined in claim 1, or at least one of their addition salts with pharmaceutically acceptable mineral or organic acids, is used as active ingredient in a form intended for this use.

7. Process according to claim 6, characterized in that the compound of formula (I) is chosen from:
   - (16alpha) (±) 17-methyl-17-aza-20,21-dinoreburnamenin-14-(15H)-one,
   - (16alpha) (±) 17-propyl-17-aza-20,21-dinoreburnamenin-14-(15H)-one,
   - (16alpha) (±) 17-aza-20,21-dinoreburnamenine,
   - (16alpha) (±) 14,15-dihydro-17-methyl-17-aza-20,21-dinoreburnamenine, and their addition salts with acids.

8. As industrial products, the compounds of formulae (III), (IV') corresponding to formula (IV) in which when one of $R_1$ or $R_2$ represents a hydrogen atom, the other of $R_1$ or $R_2$ does not represent a methoxy radical, the compounds (V), (VI), (VII), (VIII) and (IX).

**Patentansprüche**
**Patentansprüche für folgende Vertragsstaaten : AT, BE, DE, FR, GB, IT, LI, LU, NL, SE**

1. Verbindungen der Formel (I)

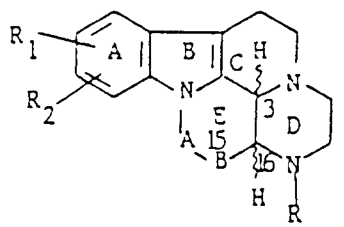

(I)

worin $R_1$ und $R_2$, die gleich oder voneinander verschieden sind, ein Wasserstoffatom, ein Halogenatom, einen Alkylrest mit 1 bis 5 Kohlenstoffatomen, einen Alkoxyrest mit 1 bis 5 Kohlenstoffatomen, einen Hydroxy-, Trifluormethyl- oder Nitrorest bedeuten, R für ein Wasserstoffatom, einen Alkylrest mit 1 bis 5 Kohlenstoffatomen, einen Aralkylrest mit 7 bis 12 Kohlenstoffatomen, einen Benzoyl- oder Alkylcarbonylrest, in dem der Alkylrest 1 bis 12 Kohlenstoffatome aufweist, steht und worin die Gruppe

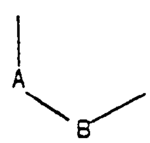

entweder

i) 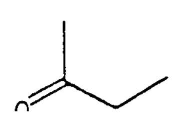

oder

ii) 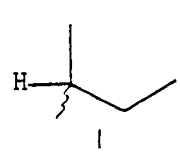

oder

iii) 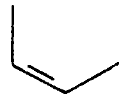

oder

iiii) 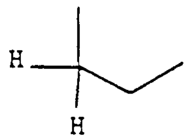

bedeutet, wobei die Produkte der Formel (I) in sämtlichen ihrer möglichen racemischen oder optisch aktiven isomeren Formen vorliegen können, sowie die Additionssalze der Produkte der Formel (I) mit Mineral- oder organischen Säuren.

**2.** Verbindungen der Formel (I), wie in Anspruch 1 definiert, dadurch gekennzeichnet, daß $R_1$ und $R_2$ ein Wasserstoffatom, einen Methyl-, Ethyl-, Methoxy- oder Ethoxyrest, ein Chloratom, eine Hydroxy-, Trifluormethyl- oder Nitrogruppe darstellen und R für ein Wasserstoffatom, einen Methyl-, Ethyl- oder Propyl-, Benzyl, Phenethyl- oder Benzoylrest steht, in sämtlichen ihrer möglichen, racemischen oder optisch aktiven isomeren Formen sowie deren Additionssalze mit Mineral- oder organischen Säuren.

**3.** Verbindungen der Formel (I), wie in den Ansprüchen 1 und 2 definiert, dadurch gekennzeichnet, daß $R_2$ für ein Wasserstoffatom steht, $R_1$ ein Wasserstoffatom, einen Methoxy- oder Ethoxyrest, ein Chloratom bedeutet, in sämtlichen ihrer möglichen racemischen oder optisch aktiven isomeren Formen sowie deren Additionssalze mit Mineral- oder organischen Säuren.

**4.** Verbindungen der Formel (I), wie in den Ansprüchen 1 bis 3 definiert, dadurch gekennzeichnet, daß das Wasserstoffatom in 3-Stellung und das Wasserstoffatom in 16-Stellung trans-ständig sind, in sämtlichen ihrer möglichen racemischen oder optisch aktiven isomeren Formen sowie deren Additionssalze mit Mineral- oder organischen Säuren.

**5.** (16α)-(±)-17-Methyl-17-aza-20,21-dinoreburnamenin-14(15H)-on,
(16α)-(±)-17-Propyl-17-aza-20,21-dinoreburnamenin-14(15H)-on,
(16α)-(±)-17-aza-20,21-dinoreburnamenin,
(16α)-(±)-14,15-Dihydro-17-methyl-17-aza-20,21-dinoreburnamenin
und deren Additionssalze mit Säuren.

**6.** Verfahren zur Herstellung der Verbindungen der Formel (I), wie in Anspruch 1 definiert, dadurch gekennzeichnet, daß man eine Verbindung der Formel (II)

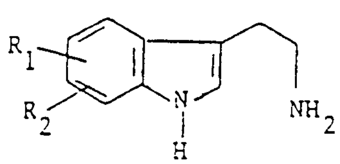

(II)

worin $R_1$ und $R_2$ die vorstehend angegebene Bedeutung besitzen, mit Chloracetonitril derart umsetzt, daß man zu einer Verbindung der Formel (III)

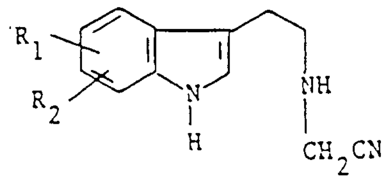

(III)

gelangt, die man zu einer Verbindung der Formel (IV)

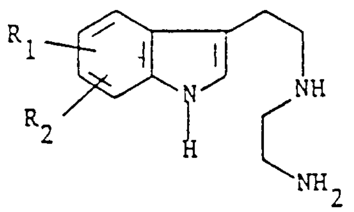

(IV)

reduziert, welche man mit einem Alkylmaleat oder -fumarat der Formel

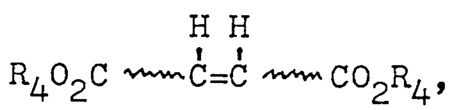

worin $R_4$ für einen Alkylrest mit 1 bis 4 Kohlenstoffatomen steht, kondensiert, um zu einer Verbindung der Formel (V)

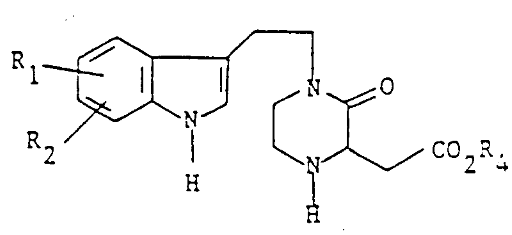

(V)

zu gelangen, die man mit einem Reagens reagieren läßt, welches in der Lage ist, einen Rest R' einzuführen, wobei R' die vorstehend für R mit Ausnahme der Bedeutung Wasserstoff angegebenen Bedeutungen besitzt, um zu einer Verbindung der Formel (VI)

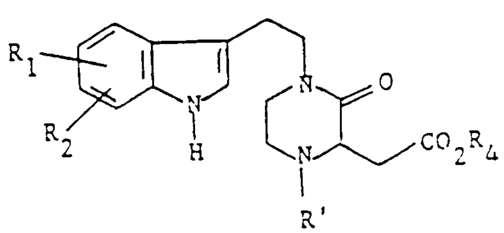

(VI)

zu gelangen, welche man mit einem Cyclisierungsmittel umsetzt, um eine Verbindung der Formel (VII)

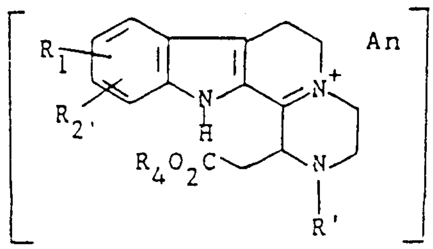

(VII)

zu erhalten, worin $R_1$, $R_2$, $R_4$ und R' die vorstehend angegebenen Bedeutungen besitzen und An ein sich von einer starken Säure ableitendes Anion wiedergibt, und daß man entweder die Verbindung der Formel (VII) zu einer Verbindung der Formel (VIII), worin die beiden Wasserstoffatome in 3- und 16-Stellung in cis-Stellung vorliegen

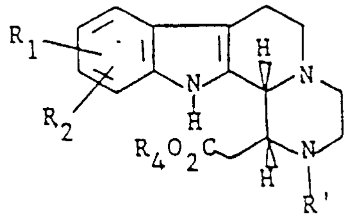

(VIII)

reduziert, von der man gegebenenfalls die Gruppe R' im Falle, daß diese einen Benzoyl- oder Alkylcarbonylrest bedeutet, entfernt, und welche man cyclisiert, um eine einem Produkt der Formel (I) entsprechende Verbindung der Formel

$$(I_{A_{1b}})$$

zu erhalten, worin

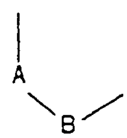

für

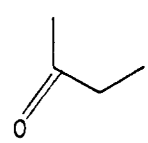

steht, die beiden Wasserstoffatome in 3- und 16-Stellung in cis-Stellung vorliegen und R ein Wasserstoffatom bedeutet, und die Verbindung der Formel

$$(I_{A_{1b}})$$

gegebenenfalls der Einwirkung eines Reagens unterzieht, das in der Lage ist, einen Rest R' einzuführen, wobei R' die für R mit Ausnahme der Bedeutung Wasserstoff angegebenen Bedeutungen besitzt,

um eine Verbindung der Formel

$$(I_{A_{1c}})$$

zu erhalten, worin R die vorstehend für R' angegebene Bedeutung besitzt, oder die Verbindung der Formel (VII) zu einer Verbindung der Formel (IX)

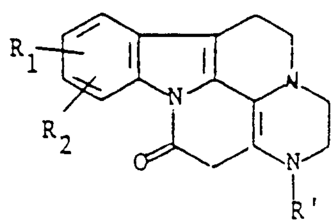

(IX)

cyclisiert, welche man reduziert, um eine einem Produkt der Formel (I) entsprechende Verbindung der Formel

$$(I_{A_{2a}})$$

zu erhalten, worin

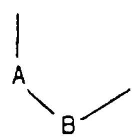

für

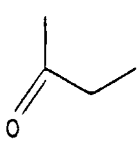

steht,
die beiden Wasserstoffatome in 3- und 16-Stellung sich in trans-Stellung befinden und R die vorstehend für R' angegebene Bedeutung besitzt, von der man gegebenenfalls die Gruppe R' im Falle, daß diese einen Benzoyl- oder Alkylcarbonylrest bedeutet, entfernt, um eine Verbindung der Formel

$$(I_{A_{2b}})$$

zu erhalten, worin R für ein Wasserstoffatom steht, welche man gegebenenfalls der Einwirkung eines Reagens unterzieht, das in der Lage ist, einen Rest R' einzuführen, wobei R' die für R angegebenen Bedeutungen mit Ausnahme der Bedeutung Wasserstoff besitzt, um eine Verbindung der Formel

$$(I_{A_{2c}})$$

zu erhalten, worin R die vorstehend für R' angegebene Bedeutung besitzt, wobei die Verbindungen der Formeln

$$(I_{A_{1b}}),\ (I_{A_{1c}}),\ (I_{A_{2a}}),$$

$$(I_{A_{2b}}) \text{ und } (I_{A_{2c}})$$

gewünschtenfalls zu entsprechenden Verbindungen der Formeln

$$(I_{B_{1b}}),\ (I_{B_{1c}}),\ (I_{B_{2a}}),\ (I_{B_{2b}})$$

$$\text{und } (I_{B_{2c}})\ ,$$

die den Verbindungen der Formel (I) entsprechen, worin

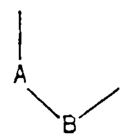

für

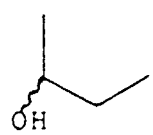

steht, reduziert werden, wobei die Verbindungen der Formeln

$$(I_{B_{1b}}),\ (I_{B_{1c}}),\ (I_{B_{2a}}),\ (I_{B_{2b}}),\ (I_{B_{2c}})$$

gewünschtenfalls der Einwirkung eines Dehydratationsmittels unterzogen werden, um die entsprechenden Verbindungen der Formeln

$$(I_{C_{1b}}),\ (I_{C_{1c}}),\ (I_{C_{2a}}),\ (I_{C_{2b}}) \text{ und } (I_{C_{2c}}),$$

die den Verbindungen der Formel (I) entsprechen, worin

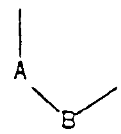

für

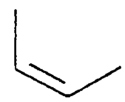

steht,
zu erhalten, wobei die Verbindungen der Formeln

$(I_{C_{1b}})$,

$(I_{C_{1c}})$, $(I_{C_{2a}})$, $(I_{C_{2b}})$ und $(I_{C_{2c}})$

gewünschtenfalls der Einwirkung eines Reduktionsmittels unterzogen werden, um die entsprechenden Verbindungen der Formeln

$(I_{D_{1b}})$,

$(I_{D_{1c}})$, $(I_{D_{2a}})$, $(I_{D_{2b}})$ und $(I_{D_{2c}})$,

die die Verbindungen der Formel (I) wiedergeben, worin

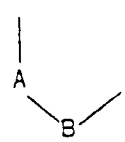

für

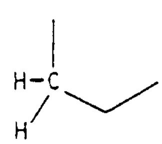

steht, zu erhalten, und gewünschtenfalls sämtliche erhaltenen Produkte der Formel (I) mit einer Mineral- oder organischen Säure behandelt, um hieraus die Salze zu bilden.

7. Als Arzneimittel die Verbindungen der Formel (I) gemäß Anspruch 1, wobei die Produkte der Formel (I) in sämtlichen ihrer möglichen racemischen oder optisch aktiven isomeren Formen vorliegen können, sowie deren Additionssalze mit pharmazeutisch verträglichen Mineral- oder organischen Säuren.

8. Als Arzneimittel die Verbindungen,wie in den Ansprüchen 2 bis 4 definiert.

9. Als Arzneimittel die Verbindungen des Anspruchs 5.

10. Pharmazeutische Zusammensetzungen, enthaltend als Wirkstoff zumindest eines der Arzneimittel, wie in einem der Ansprüche 7 bis 9 definiert.

11. Als industrielle Produkte die Verbindungen der Formeln (III), (IV'), entsprechend der Formel (IV), worin, wenn eines von $R_1$ oder $R_2$ ein Wasserstoffatom bedeutet, das andere von $R_1$ oder $R_2$ keinen Methoxyrest darstellt, die Verbindungen (V), (VI), (VII), (VIII) und (IX).

**Patentansprüche für folgenden Vertragsstaat: ES**

1. Verfahren zur Herstellung von Verbindungen der Formel (I)

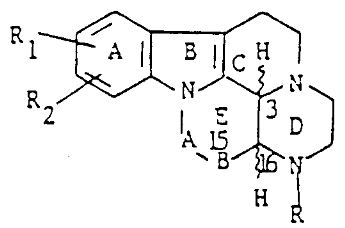

(I)

worin $R_1$ und $R_2$, die gleich oder voneinander verschieden sind, ein Wasserstoffatom, ein Halogenatom, einen Alkylrest mit 1 bis 5 Kohlenstoffatomen, einen Alkoxyrest mit 1 bis 5 Kohlenstoffatomen, einen Hydroxy-, Trifluormethyl- oder Nitrorest bedeuten, R für ein Wasserstoffatom, einen Alkylrest mit 1 bis 5 Kohlenstoffatomen, einen Aralkylrest mit 7 bis 12 Kohlenstoffatomen, einen Benzoyl- oder Alkylcarbonylrest, in dem der Alkylrest 1 bis 12 Kohlenstoffatome aufweist, steht und worin die Gruppe

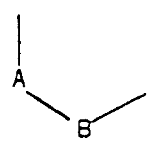

entweder

i) 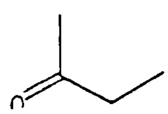

oder

ii) 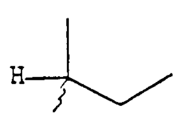

oder

iii) 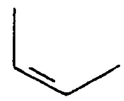

oder

iiii) 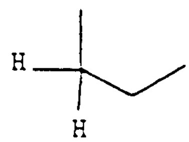

bedeutet, wobei die Produkte der Formel (I) in sämtlichen ihrer möglichen racemischen oder optisch aktiven isomeren Formen vorliegen können, sowie von den Additionssalzen der Produkte der Formel (I) mit Mineral- oder organischen Säuren,
dadurch gekennzeichnet, daß man eine Verbindung der Formel (II)

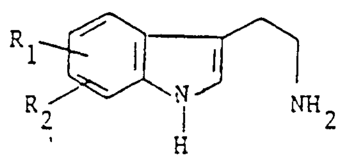

(II)

worin $R_1$ und $R_2$ die vorstehend angegebene Bedeutung besitzen, mit Chloracetonitril derart umsetzt, daß man zu einer Verbindung der Formel (III)

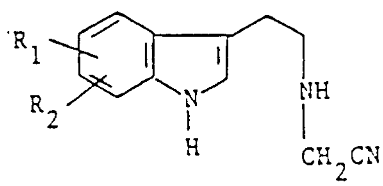

(III)

gelangt, die man zu einer Verbindung der Formel (IV)

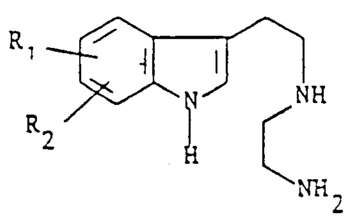

(IV)

reduziert, welche man mit einem Alkylmaleat oder -fumarat der Formel

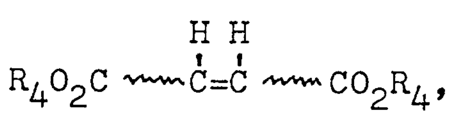

worin $R_4$ für einen Alkylrest mit 1 bis 4 Kohlenstoffatomen steht, kondensiert, um zu einer Verbindung der Formel (V)

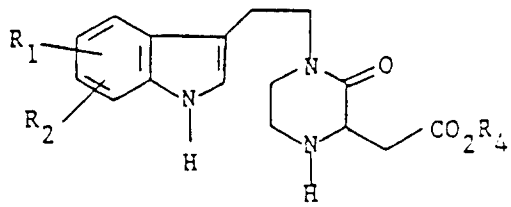

(V)

zu gelangen, die man mit einem Reagens reagieren läßt, welches in der Lage ist, einen Rest R' einzuführen, wobei R' die vorstehend für R mit Ausnahme der Bedeutung Wasserstoff angegebenen Bedeutungen besitzt, um zu einer Verbindung der Formel (VI)

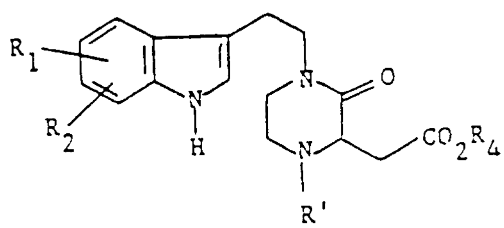

(VI)

zu gelangen, welche man mit einem Cyclisierungsmittel umsetzt, um eine Verbindung der Formel (VII)

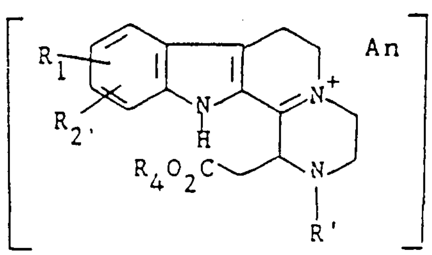

(VII)

zu erhalten, worin $R_1$, $R_2$, $R_4$ und R' die vorstehend angegebenen Bedeutungen besitzen und An ein sich von einer starken Säure ableitendes Anion wiedergibt, und daß man entweder die Verbindung der Formel (VII) zu einer Verbindung der Formel (VIII), worin die beiden Wasserstoffatome in 3- und 16-Stellung in cis-Stellung vorliegen

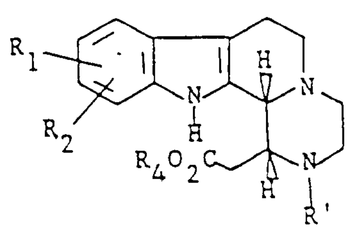

(VIII)

reduziert, von der man gegebenenfalls die Gruppe R' im Falle, daß diese einen Benzoyl- oder Alkylcarbonylrest bedeutet, entfernt, und welche man cyclisiert, um eine einem Produkt der Formel (I) entsprechende Verbindung der Formel

$$(I_{A_{1b}})$$

zu erhalten, worin

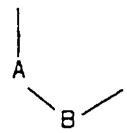

für

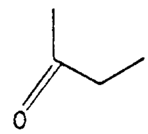

steht,
die beiden Wasserstoffatome in 3- und 16-Stellung in cis-Stellung vorliegen und R ein Wasserstoffatom bedeutet, und die Verbindung der Formel

$(I_{A_{1b}})$

gegebenenfalls der Einwirkung eines Reagens unterzieht, das in der Lage ist, einen Rest R' einzuführen, wobei R' die für R mit Ausnahme der Bedeutung Wasserstoff angegebenen Bedeutungen besitzt, um eine Verbindung der Formel

$(I_{A_{1c}})$

zu erhalten, worin R die vorstehend für R' angegebene Bedeutung besitzt, oder die Verbindung der Formel (VII) zu einer Verbindung der Formel (IX)

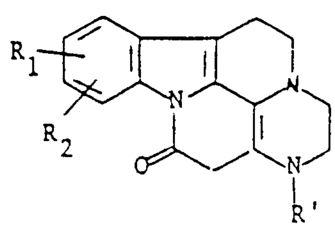

(IX)

cyclisiert, welche man reduziert, um eine einem Produkt der Formel (I) entsprechende Verbindung der Formel

$(I_{A_{2a}})$

zu erhalten, worin

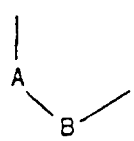

für

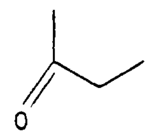

steht, die beiden Wasserstoffatome in 3- und 16-Stellung sich in trans-Stellung befinden und R die vorstehend für R' angegebene Bedeutung besitzt, von der man gegebenenfalls die Gruppe R' im Falle, daß diese einen Benzoyl- oder Alkylcarbonylrest bedeutet, entfernt, um eine Verbindung der Formel

$$(I_{A_{2b}})$$

zu erhalten, worin R für ein Wasserstoffatom steht, welche man gegebenenfalls der Einwirkung eines Reagens unterzieht, das in der Lage ist, einen Rest R' einzuführen, wobei R' die für R angegebenen Bedeutungen mit Ausnahme der Bedeutung Wasserstoff besitzt, um eine Verbindung der Formel

$$(I_{A_{2c}})$$

zu erhalten, worin R die vorstehend für R' angegebene Bedeutung besitzt. wobei die Verbindungen der Formeln

$$(I_{A_{1b}}),\ (I_{A_{1c}}),\ (I_{A_{2a}}),$$

$$(I_{A_{2b}}) \text{ und } (I_{A_{2c}})$$

gewünschtenfalls zu entsprechenden Verbindungen der Formeln

$$(I_{B_{1b}}),\ (I_{B_{1c}}),\ (I_{B_{2a}}),\ (I_{B_{2b}})$$

$$\text{und } (I_{B_{2c}}),$$

die den Verbindungen der Formel (I) entsprechen, worin

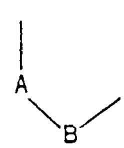

für

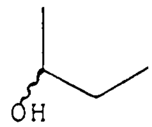

steht, reduziert werden, wobei die Verbindungen der Formeln

$(I_{B_{1b}})$, $(I_{B_{1c}})$, $(I_{B_{2a}})$, $(I_{B_{2b}})$, $(I_{B_{2c}})$

gewünschtenfalls der Einwirkung eines Dehydratationsmittels unterzogen werden, um die entsprechenden Verbindungen der Formeln

$(I_{C_{1b}})$, $(I_{C_{1c}})$, $(I_{C_{2a}})$, $(I_{C_{2b}})$ und $(I_{C_{2c}})$,

die den Verbindungen der Formel (I) entsprechen, worin

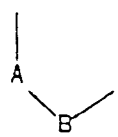

für

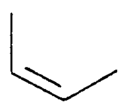

steht,
zu erhalten, wobei die Verbindungen der Formeln

$(I_{C_{1b}})$,

$(I_{C_{1c}})$, $(I_{C_{2a}})$, $(I_{C_{2b}})$ und $(I_{C_{2c}})$

gewünschtenfalls der Einwirkung eines Reduktionsmittels unterzogen werden, um die entsprechenden Verbindungen der Formeln

$(I_{D_{1b}})$,

$(I_{D_{1c}})$, $(I_{D_{2a}})$, $(I_{D_{2b}})$ und $(I_{D_{2c}})$,

die die Verbindungen der Formel (I) wiedergeben, worin

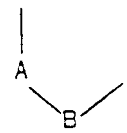

für

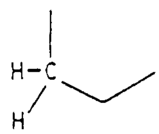

steht,
zu erhalten, und gewünschtenfalls sämtliche erhaltenen Produkte der Formel (I) mit einer Mineral- oder organischen Säure behandelt, um hieraus die Salze zu bilden.

**2.** Verfahren gemäß Anspruch 1, dadurch gekennzeichnet, daß man von einer Verbindung der Formel (II), worin $R_1$ und $R_2$ ein Wasserstoffatom, einen Methyl-, Ethyl-, Methoxy- oder Ethoxyrest, ein Chloratom, eine Hydroxy-, Trifluormethyl- oder Nitrogruppe bedeuten, und einem Reagens, das in der Lage ist, einen Rest R' einzuführen, derart, daß R' einen Methyl-, Ethyl- oder Propyl-, Benzyl-, Phenethyl- oder Benzoylrest bedeutet, ausgeht.

**3.** Verfahren gemäß Anspruch 1 oder 2, dadurch gekennzeichnet, daß man Verbindungen der Formel (I), worin $R_2$ für ein Wasserstoffatom steht, $R_1$ ein Wasserstoffatom, einen Methoxy- oder Ethoxyrest, ein Chloratom bedeutet, in sämtlichen ihrer möglichen racemischen oder optisch aktiven isomeren Formen sowie deren Additionssalze mit Mineral- oder organischen Säuren herstellt.

**4.** Verfahren gemäß einem der Ansprüche 1 bis 3, dadurch gekennzeichnet, daß man Verbindungen der Formel (I), worin das Wasserstoffatom in 3-Stellung und das Wasserstoffatom in 16-Stellung trans-ständig sind, in sämtlichen ihrer möglichen racemischen oder optisch aktiven isomeren Formen sowie deren Additionssalze mit Mineral- oder organischen Säuren herstellt.

**5.** Verfahren gemäß Anspruch 1, dadurch gekennzeichnet, daß man herstellt:
(16α)-(±)-17-Methyl-17-aza-20,21-dinoreburnamenin-14(15H)-on,
(16α)-(±)-17-Propyl-17-aza-20,21-dinoreburnamenin-14(15H)-on,
(16α)-(±)-17-aza-20,21-dinoreburnamenin,
(16α)-(±)-14,15-Dihydro-17-methyl-17-aza-20,21-dinoreburnamenin
und deren Additionssalze mit Säuren.

**Patentansprüche für folgenden Vertragsstaat : GR**

**1.** Verfahren zur Herstellung von Verbindungen der Formel (I)

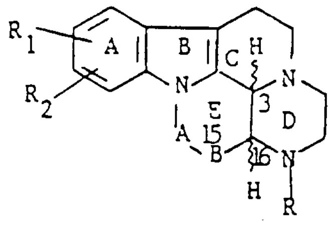

(I)

worin $R_1$ und $R_2$, die gleich oder voneinander verschieden sind, ein Wasserstoffatom, ein Halogenatom, einen Alkylrest mit 1 bis 5 Kohlenstoffatomen, einen Alkoxyrest mit 1 bis 5 Kohlenstoffatomen, einen Hydroxy-, Trifluormethyl- oder Nitrorest bedeuten, R für ein Wasserstoffatom, einen Alkylrest mit 1 bis 5 Kohlenstoffatomen, einen Aralkylrest mit 7 bis 12 Kohlenstoffatomen, einen Benzoyl- oder Alkylcarbonylrest, in dem der Alkylrest 1 bis 12 Kohlenstoffatome aufweist, steht und worin die Gruppe

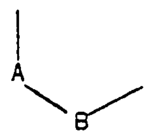

entweder

i) 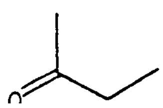

oder

ii) 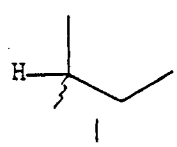

oder

iii) 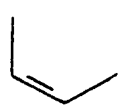

oder

iiii) 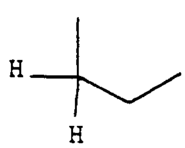

bedeutet, wobei die Produkte der Formel (I) in sämtlichen ihrer möglichen racemischen oder optisch

aktiven isomeren Formen vorliegen können, sowie von den Additionssalzen der Produkte der Formel (I) mit Mineral- oder organischen Säuren,
dadurch gekennzeichnet, daß man eine Verbindung der Formel (II)

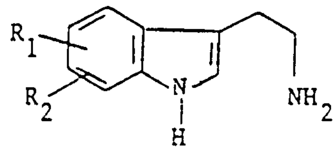

(II)

worin $R_1$ und $R_2$ die vorstehend angegebene Bedeutung besitzen, mit Chloracetonitril derart umsetzt, daß man zu einer Verbindung der Formel (III)

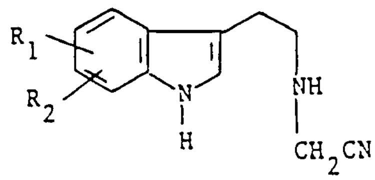

(III)

gelangt, die man zu einer Verbindung der Formel (IV)

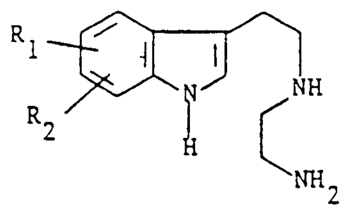

(IV)

reduziert, welche man mit einem Alkylmaleat oder -fumarat der Formel

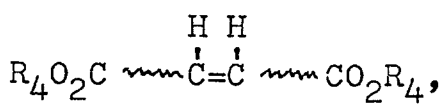

worin $R_4$ für einen Alkylrest mit 1 bis 4 Kohlenstoffatomen steht, kondensiert, um zu einer Verbindung der Formel (V)

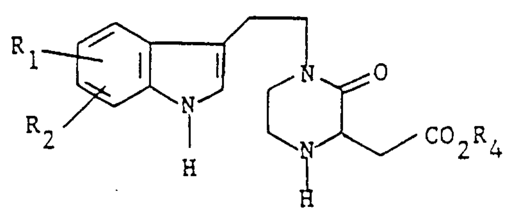

(V)

zu gelangen, die man mit einem Reagens reagieren läßt, welches in der Lage ist, einen Rest R' einzuführen, wobei R' die vorstehend für R mit Ausnahme der Bedeutung Wasserstoff angegebenen Bedeutungen besitzt, um zu einer Verbindung der Formel (VI)

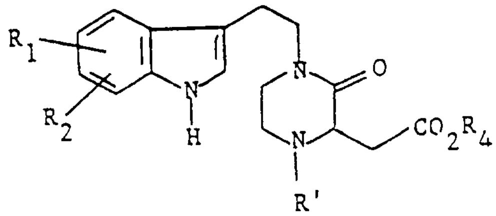

(VI)

zu gelangen, welche man mit einem Cyclisierungsmittel umsetzt, um eine Verbindung der Formel (VII)

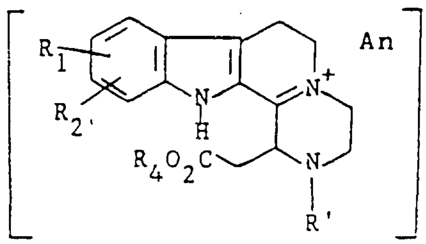

(VII)

zu erhalten, worin $R_1$, $R_2$, $R_4$ und R' die vorstehend angegebenen Bedeutungen besitzen und An ein sich von einer starken Säure ableitendes Anion wiedergibt, und daß man entweder die Verbindung der Formel (VII) zu einer Verbindung der Formel (VIII), worin die beiden Wasserstoffatome in 3- und 16-Stellung in cis-Stellung vorliegen

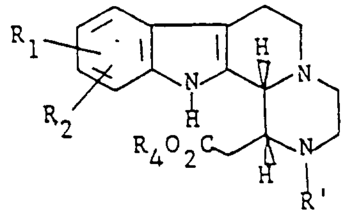

(VIII)

reduziert, von der man gegebenenfalls die Gruppe R' im Falle, daß diese einen Benzoyl- oder Alkylcarbonylrest bedeutet, entfernt, und welche man cyclisiert, um eine einem Produkt der Formel (I) entsprechende Verbindung der Formel

$$(I_{A_{1b}})$$

zu erhalten, worin

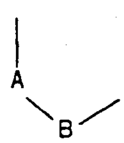

für

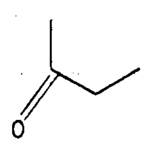

steht,
die beiden Wasserstoffatome in 3- und 16-Stellung in cis-Stellung vorliegen und R ein Wasserstoffatom bedeutet, und die Verbindung der Formel

$$(I_{A_{1b}})$$

gegebenenfalls der Einwirkung eines Reagens unterzieht, das in der Lage ist, einen Rest R' einzuführen, wobei R' die für R mit Ausnahme der Bedeutung Wasserstoff angegebenen Bedeutungen besitzt, um eine Verbindung der Formel

$$(I_{A_{1c}})$$

zu erhalten, worin R die vorstehend für R' angegebene Bedeutung besitzt, oder die Verbindung der Formel (VII) zu einer Verbindung der Formel (IX)

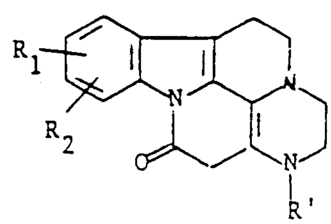

(IX)

cyclisiert, welche man reduziert, um eine einem Produkt der Formel (I) entsprechende Verbindung der Formel

$$(I_{A_{2a}})$$

zu erhalten, worin

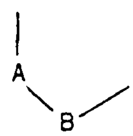

für

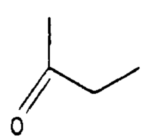

steht,
die beiden Wasserstoffatome in 3- und 16-Stellung sich in trans-Stellung befinden und R die vorstehend für R' angegebene Bedeutung besitzt, von der man gegebenenfalls die Gruppe R' im Falle, daß diese einen Benzoyl- oder Alkylcarbonylrest bedeutet, entfernt, um eine Verbindung der Formel

$$(I_{A_{2b}})$$

zu erhalten, worin R für ein Wasserstoffatom steht, welche man gegebenenfalls der Einwirkung eines Reagens unterzieht, das in der Lage ist, einen Rest R' einzuführen, wobei R' die für R angegebenen Bedeutungen mit Ausnahme der Bedeutung Wasserstoff besitzt, um eine Verbindung der Formel

$$(I_{A_{2c}})$$

zu erhalten, worin R die vorstehend für R' angegebene Bedeutung besitzt, wobei die Verbindungen der Formeln

$$(I_{A_{1b}}),\ (I_{A_{1c}}),\ (I_{A_{2a}}),$$

$$(I_{A_{2b}})\ \text{und}\ (I_{A_{2c}})$$

gewünschtenfalls zu entsprechenden Verbindungen der Formeln

$$(I_{B_{1b}}),\ (I_{B_{1c}}),\ (I_{B_{2a}}),\ (I_{B_{2b}})$$

$$\text{und}\ (I_{B_{2c}})\ ,$$

die den Verbindungen der Formel (I) entsprechen, worin

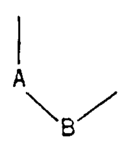

für

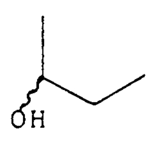

steht, reduziert werden, wobei die Verbindungen der Formeln

$$(I_{B_{1b}}),\ (I_{B_{1c}}),\ (I_{B_{2a}}),\ (I_{B_{2b}}),\ (I_{B_{2c}})$$

gewünschtenfalls der Einwirkung eines Dehydratationsmittels unterzogen werden, um die entsprechenden Verbindungen der Formeln

$$(I_{C_{1b}}),\ (I_{C_{1c}}),\ (I_{C_{2a}}),\ (I_{C_{2b}})\ \text{und}\ (I_{C_{2c}}),$$

die den Verbindungen der Formel (I) entsprechen, worin

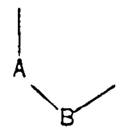

für

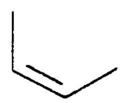

steht,
zu erhalten, wobei die Verbindungen der Formeln

$$(I_{C_{1b}}),$$

$$(I_{C_{1c}}),\ (I_{C_{2a}}),\ (I_{C_{2b}})\ \text{und}\ (I_{C_{2c}})$$

gewünschtenfalls der Einwirkung eines Reduktionsmittels unterzogen werden, um die entsprechenden Verbindungen der Formeln

$$(I_{D_{1b}}),$$

$$(I_{D_{1c}}),\ (I_{D_{2a}}),\ (I_{D_{2b}})\ \text{und}\ (I_{D_{2c}}),$$

die die Verbindungen der Formel (I) wiedergeben, worin

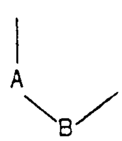

für

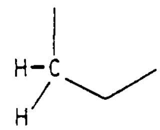

steht, zu erhalten, und gewünschtenfalls sämtliche erhaltenen Produkte der Formel (I) mit einer Mineral- oder organischen Säure behandelt, um hieraus die Salze zu bilden.

**2.** Verfahren gemäß Anspruch 1, dadurch gekennzeichnet, daß man von einer Verbindung der Formel (II), Worin $R_1$ und $R_2$ ein Wasserstoffatom, einen Methyl-, Ethyl-, Methoxy- oder Ethoxyrest, ein Chloratom, eine Hydroxy-, Trifluormethyl- oder Nitrogruppe bedeuten, und einem Reagens, das in der Lage ist, einen Rest R' einzuführen, derart, daß R' einen Methyl-, Ethyl- oder Propyl-, Benzyl-, Phenethyl- oder Benzoylrest bedeutet, ausgeht.

**3.** Verfahren gemäß Anspruch 1 oder 2, dadurch gekennzeichnet, daß man Verbindungen der Formel (I), Worin $R_2$ für ein Wasserstoffatom steht, $R_1$ ein Wasserstoffatom, einen Methoxy- oder Ethoxyrest, ein Chloratom bedeutet, in sämtlichen ihrer möglichen racemischen oder optisch aktiven isomeren Formen sowie deren Additionssalze mit Mineral- oder organischen Säuren herstellt.

**4.** Verfahren gemäß einem der Ansprüche 1 bis 3, dadurch gekennzeichnet, daß man Verbindungen der Formel (I), worin das Wasserstoffatom in 3-Stellung und das Wasserstoffatom in 16-Stellung trans-ständig sind, in sämtlichen ihrer möglichen racemischen oder optisch aktiven isomeren Formen sowie deren Additionssalze mit Mineral- oder organischen Säuren herstellt.

**5.** Verfahren gemäß Anspruch 1, dadurch gekennzeichnet, daß man herstellt:
(16α)-(±)-17-Methyl-17-aza-20,21-dinoreburnamenin-14(15H)-on,
(16α)-(±)-17-Propyl-17-aza-20,21-dinoreburnamenin-14(15H)-on,
(16α)-(±)-17-aza-20,21-dinoreburnamenin,
(16α)-(±)-14,15-Dihydro-17-methyl-17-aza-20,21-dinoreburnamenin
und deren Additionssalze mit Säuren.

**6.** Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer Additionssalze mit pharmazeutisch verträglichen Mineral- oder organischen Säuren in eine für diese Verwendung bestimmte Form überführt.

**7.** Verfahren gemäß Anspruch 6, dadurch gekennzeichnet, daß die Verbindung der Formel (I) ausgewählt wird unter

(16α)-(±)-17-Methyl-17-aza-20,21-dinoreburnamenin-14(15H)-on,
(16α)-(±)-17-Propyl-17-aza-20,21-dinoreburnamenin-14(15H)-on,
(16α)-(±)-17-aza-20,21-dinoreburnamenin,
(16α)-(±)-14,15-Dihydro-17-methyl-17-aza-20,21-dinoreburnamenin

und ihren Additionssalzen mit Säuren.

**8.** Als industrielle Produkte die Verbindungen der Formeln (III), (IV'), entsprechend der Formel (IV), worin, wenn eines von $R_1$ oder $R_2$ ein Wasserstoffatom bedeutet, das andere von $R_1$ oder $R_2$ keine Methoxygruppe wiedergibt, die Verbindungen (V), (VI), (VII), (VIII) und (IX).